# EUROPEAN PATENT SPECIFICATION

(11) **EP 2 080 805 B1**
(45) Date of publication and mention of the grant of the patent: **08.10.2014**
(21) Application number: 09156877.4
(22) Date of filing: 18.08.1999
(51) Int. Cl.: C12N 15/82, C12N 5/10, A01H 5/00

(54) **Plant expression vectors**
Pflanzliche Expressionsvektoren
Vecteur d'expression de plante

(30) Priority: 19.08.1998 US 97150 P
(43) Date of publication of application: 22.07.2009
(62) Divisional of application: 99942392.4
(73) Proprietor: Monsanto Technology LLC, St. Louis, MO 63167 (US)
(72) Inventor: Conner, Timothy W., St. Louis, MO 63167 (US); Santino, Colleen G., St. Louis, MO 63116 (US)
(74) Representative: Von Kreisler Selting Werner - Partnerschaft von Patentanwälten und Rechtsanwälten mbB

(56) References cited:
- WO-A-93/19189
- WO-A-95/06742
- MCELWAIN E F AND SPIKER S: "A wheat cDNA clone which is homologous to the 17 kd heat-shock protein gene family of soybean" NUCLEIC ACIDS RESEARCH, vol. 17, no. 4, 1989, page 1764, XP002138071
- KOZIEL M G ET AL: "OPTIMIZING EXPRESSION OF TRANSGENES WITH EMPHASIS ON POST-TRANSCRIPTIONAL EVENTS" PLANT MOLECULAR BIOLOGY, SPRINGER, DORDRECHT, NL, vol. 32, 1 January 1996 (1996-01-01), pages 393-405, XP002936764 ISSN: 0167-4412

<110> Monsanto Technology LLC
<120> Novel Plant Expression Vectors
<130> 091143ep
<150> EP 99942392.4
   <151> 1999-08-18
<150> 60/097150
   <151> 1998-08-19
<160> 63
<170> PatentIn Ver. 2.1
<210> 1
   <211> 36
   <212> DNA
   <213> Artificial Sequence
<220>
   <223> synthetic
<400> 1 gatggatcct ctagaaccat cttccacaca ctcaag 36
<210> 2
   <211> 34
   <212> DNA
   <213> Artificial Sequence
<220>
   <223> synthetic
<400> 2 gatccatggc gcagatctta tggtgtgttg tccc 34
<210> 3
   <211> 13
   <212> DNA
   <213> Artificial Sequence
<220>
   <223> synthetic
<400> 3 gatccaaggg agg 13
<210> 4
   <211> 9
   <212> DNA
   <213> Artificial Sequence
<220>
   <223> synthetic
<400> 4 cctcccttg 9
<210> 5
   <211> 48
   <212> DNA
   <213> Artificial Sequence
<220>
   <223> synthetic
<400> 5 gatcctctag agaattccct tttcctacct acgatccgat accgaatt 48
<210> 6
   <211> 37
   <212> DNA
   <213> Artificial Sequence
<220>
   <223> synthetic
<400> 6 ttccgagcgc acaagccaaa ccaaagcaag atctgac 37
<210> 7
   <211> 35
   <212> DNA
   <213> Artificial Sequence
<220>
   <223> synthetic
<400> 7 tcggatcgta ggtaggaaaa gggaattctc tagag 35
<210> 8
   <211> 50
   <212> DNA
   <213> Artificial Sequence
<220>
   <223> synthetic
<400> 8 catggtcaga tcttgctttg gtttggcttg tgcgctcgga aaattcggta 50
<210> 9
   <211> 49
   <212> DNA
   <213> Artificial Sequence
<220>
   <223> synthetic
<400> 9 gatcctctag agggccacca ccacggtgcg cgccaagaca aggcagggg 49
<210> 10
   <211> 31
   <212> DNA
   <213> Artificial Sequence
<220>
   <223> synthetic
<400> 10 agagaaattc gtcaatccgc agcagatctg c 31
<210> 11
   <211> 35
   <212> DNA
   <213> Artificial Sequence
<220>
   <223> synthetic
<400> 11 gtcttggcgc gcaccgtggt ggtggccctc tagag 35
<210> 12
   <211> 45
   <212> DNA
   <213> Artificial Sequence
<220>
   <223> synthetic
<400> 12 catggcagat ctgctgcgga ttgacgaatt tctctcccct gcctt 45
<210> 13
   <211> 12
   <212> DNA
   <213> Artificial Sequence
<220>
   <223> synthetic
<400> 13 gatctacggg gt 12
<210> 14
   <211> 12
   <212> DNA
   <213> Artificial Sequence
<220>
   <223> synthetic
<400> 14 ctagaccccg ta 12
<210> 15
   <211> 31
   <212> DNA
   <213> Artificial Sequence
<220>
   <223> synthetic
<400> 15 aggaattcgc tcctggccat ggagctgctt c 31
<210> 16
   <211> 55
   <212> DNA
   <213> Artificial Sequence
<220>
   <223> synthetic
<400> 16 agggatccaa aaaacacaca cagatctccg ctcacttatt catagttcac caaag 55
<210> 17
   <211> 31
   <212> DNA
   <213> Artificial Sequence
<220>
   <223> synthetic
<400> 17 aggaattctg catgcgtttg gacgtatgct c 31
<210> 18
   <211> 56
   <212> DNA
   <213> Artificial Sequence
<220>
   <223> synthetic
<400> 18 agggatccaa aaaacacaca cagatctaat tccttttttt ttgcactcaa aatcag 56
<210> 19
   <211> 32
   <212> DNA
   <213> Artificial Sequence
<220>
   <223> synthetic
<400> 19 aggaattcaa caagaacgag ggagggatac ac 32
<210> 20
   <211> 54
   <212> DNA
   <213> Artificial Sequence
<220>
   <223> synthetic
<400> 20 agggatccaa aaaacacaca cagatctctt gacctcacaa tccaattgga attc 54
<210> 21
   <211> 35
   <212> DNA
   <213> Artificial Sequence
<220>
   <223> synthetic
<400> 21 gtgtccaccc acccctcgat ctctcgctcg ccgcc 35
<210> 22
   <211> 43
   <212> DNA
   <213> Artificial Sequence
<220>
   <223> synthetic
<400> 22 gccgatcgga tcgcgtggtt ggatcatcac aactcggcaa aga 43
<210> 23
   <211> 43
   <212> DNA
   <213> Artificial Sequence
<220>
   <223> synthetic
<400> 23 cgatcggcgg cggcgagcga gagatcgagg ggtgggtgga cac 43
<210> 24
   <211> 38
   <212> DNA
   <213> Artificial Sequence
<220>
   <223> synthetic
<400> 24 gatctctttg ccgagttgtg atgatccacc acgcgatc 38
<210> 25
   <211> 56
   <212> DNA
   <213> Artificial Sequence
<220>
   <223> synthetic
<400> 25 gatcctctag aaccaccaca ccactccacc agtaagaagt gcagcaggta gctagt 56
<210> 26
   <211> 39
   <212> DNA
   <213> Artificial Sequence
<220>
   <223> synthetic
<400> 26 aagccggcgt agctttgctc ttgcagctag agatctaac 39
<210> 27
   <211> 42
   <212> DNA
   <213> Artificial Sequence
<220>
   <223> synthetic
<400> 27 tgctgcactt cttactggtg gagtggtgtg gtggttctag ag 42
<210> 28
   <211> 53
   <212> DNA
   <213> Artificial Sequence
<220>
   <223> synthetic
<400> 28 catggttaga tctctagctg caagagcaaa gctacgccgg cttactagct acc 53
<210> 29
   <211> 32
   <212> DNA
   <213> Artificial Sequence
<220>
   <223> synthetic
<400> 29 tagtagagat ctcctgtttc aggtaagaga tc 32
<210> 30
   <211> 32
   <212> DNA
   <213> Artificial Sequence
<220>
   <223> synthetic
<400> 30 tagtagtcta gaagttgaat ccctgcatca tc 32
<210> 31
   <211> 29
   <212> DNA
   <213> Artificial Sequence
<220>
   <223> synthetic
<400> 31 tagtagagat ctgagctcat caggtgagg 29
<210> 32
   <211> 32
   <212> DNA
   <213> Artificial Sequence
<220>
   <223> synthetic
<400> 32 tagtagtcta gaccgggatt gaggaatctg cc 32
<210> 33
   <211> 31
   <212> DNA
   <213> Artificial Sequence
<220>
   <223> synthetic
<400> 33 tagtagagat ctccaccatt gggtatgttg c 31
<210> 34
   <211> 35
   <212> DNA
   <213> Artificial Sequence
<220>
   <223> synthetic
<400> 34 tagtagtcta gaatttcagg aactgcaaag aaagg 35
<210> 35
   <211> 31
   <212> DNA
   <213> Artificial Sequence
<220>
   <223> synthetic
<400> 35 tagtaggaat tcgttggcaa tgcggataaa g 31
<210> 36
   <211> 33
   <212> DNA
   <213> Artificial Sequence
<220>
   <223> synthetic
<400> 36 tagtaggcat gcccataaga taagggaggg ttg 33
<210> 37
   <211> 29
   <212> DNA
   <213> Artificial Sequence
<220>
   <223> synthetic
<400> 37 tagtaggaat tctaaatctt attattatc 29
<210> 38
   <211> 31
   <212> DNA
   <213> Artificial Sequence
<220>
   <223> synthetic
<400> 38 tagtaggcat gctcgacaat aagtacttgt c 31
<210> 39
   <211> 31
   <212> DNA
   <213> Artificial Sequence
<220>
   <223> synthetic
<400> 39 tagtaggaat tcggtggctt ttgcttggtg g 31
<210> 40
   <211> 31
   <212> DNA
   <213> Artificial Sequence
<220>
   <223> synthetic
<400> 40 tagtaggcat gcaagatcca tatgcctata g 31
<210> 41
   <211> 40
   <212> DNA
   <213> Artificial Sequence
<220>
   <223> synthetic
<400> 41 gtgatccatc atctacaaga gatcgatcag tagtggttag 40
<210> 42
   <211> 47
   <212> DNA
   <213> Artificial Sequence
<220>
   <223> synthetic
<400> 42 gttgctgcta accactactg atcgatctct tgtagatgat ggatcac 47
<210> 43
   <211> 41
   <212> DNA
   <213> Artificial Sequence
<220>
   <223> synthetic
<400> 43 cagcaactca ctatcgaaca cggtttcagc ttacacagat a 41
<210> 44
   <211> 38
   <212> DNA
   <213> Artificial Sequence
<220>
   <223> synthetic
<400> 44 gatctatctg tgtaagctga aaccgtgttc gatagtga 38
<210> 45
   <211> 29
   <212> DNA
   <213> Artificial Sequence
<220>
   <223> synthetic
<400> 45 cacgctgaca agctgactct agcagatct 29
<210> 46
   <211> 253
   <212> DNA
   <213> Agrobacterium tumefaciens
<400> 46
   gatcgttcaa acatttggca ataaagtttc ttaagattga atcctgttgc cggtcttgcg 60
   atgattatca tataatttct gttgaattac gttaagcatg taataattaa catgtaatgc 120
   atgacgttat ttatgagatg ggtttttatg attagagtcc cgcaattata catttaatac 180
   gcgatagaaa acaaaatata gcgcgcaaac taggataaat tatcgcgcgc ggtgtcatct 240
   atgttactag atc                                                    253
<210> 47
   <211> 804
   <212> DNA
   <213> Zea mays
<400> 47
   accgtcttcg gtacgcgctc actccgccct ctgcctttgt tactgccacg tttctctgaa 60
   tgctctcttg tgtggtgatt gctgagagtg gtttagctgg atctagaatt acactctgaa 120
   atcgtgttct gcctgtgctg attacttgcc gtcctttgta gcagcaaaat atagggacat 180
   ggtagtacga aacgaagata gaacctacac agcaatacga gaaatgtgta atttggtgct 240
   tagcggtatt tatttaagca catgttggtg ttatagggca cttggattca gaagtttgct 300
   gttaatttag gcacaggctt catactacat gggtcaatag tatagggatt catattatag 360
   gcgatactat aataatttgt tcgtctgcag agcttattat ttgccaaaat tagatattcc 420
   tattctgttt ttgtttgtgt gctgttaaat tgttaacgcc tgaaggaata aatataaatg 480
   acgaaatttt gatgtttatc tctgctcctt tattgtgacc ataagtcaag atcagatgca 540
   cttgttttaa atattgttgt ctgaagaaat aagtactgac agtattttga tgcattgatc 600
   tgcttgtttg ttgtaacaaa atttaaaaat aaagagtttc ctttttgttg ctctccttac 660
   ctcctgatgg tatctagtat ctaccaactg acactatatt gcttctcttt acatacgtat 720
   cttgctcgat gccttctccc tagtgttgac cagtgttact cacatagtct ttgctcattt 780
   cattgtaatg cagataccaa gcgg                                        804
<210> 48
   <211> 149
   <212> DNA
   <213> Oryza sativa
<400> 48
   gatctgagct catcaggtga ggattaggat tccaaataag cgataacgtt tacctggtca 60
   ctgcgattag ttcagtttac tgtgaaattc tttggaccct tcttaattat aaatttgctt 120
   gttttctcgg cagattcctc aatgccggt                                   149
<210> 49
   <211> 128
   <212> DNA
   <213> Oryza sativa
<400> 49
   gatctcctgt ttcaggtaag agatcgccat gagttgggtt tcaggcttca gtgaactgat 60
   cgggttttgt actgagccta agagaatgat gcagtgatgc tcttgtgttt gatgatgatg 120
   cagggatt                                                          128
<210> 50
   <211> 491
   <212> DNA
   <213> Oryza sativa
<400> 50
   cctccgccgc cgccggtaac caccccgccc ctctcctctt tctttctccg tttttttttc 60
   cgtctcggtc tcgatctttg gccttggtag tttgggtggg cgagaggcgg cttcgtgcgc 120
   gcccagatcg gtgcgcggga ggggcgggat ctcgcggctg gggctctcgc cggcgtggat 180
   ccggcccgga tctcgcgggg aatggggctc tcggatgtag atctgcgatc cgccgttgtt 240
   gggggagatg atggggggtt taaaatttcc gccgtgctaa acaagatcag gaagagggga 300
   aaagggcact atggtttata tttttatata tttctgctgc ttcgtcaggc ttagatgtgc 360
   tagatctttc tttcttcttt ttgtgggtag aatttgaatc cctcagcatt gttcatcggt 420
   agtttttctt ttcatgattt gtgacaaatg cagcctcgtg cggagctttt ttgtaggtag 480
   aagtgatcaa c                                                      491
<210> 51
   <211> 1186
   <212> DNA
   <213> Oryza sativa
<400> 51
   ccaccattgg gtatgttgct tccattgcca aactgttccc ttttacccat aggctgattg 60
   atcttggctg tgtgattttt tgcttgggtt tttgagctga ttcagcggcg cttgcagcct 120
   cttgatcgtg gtcttggctc gcccatttct tgcgattctt tggtgggtcg tcagctgaat 180
   cttgcaggag tttttgctga catgttcttg ggtttactgc tttcggtaaa tctgaaccaa 240
   gaggggggtt tctgctgcag tttagtgggt ttactatgag cggattcggg gtttcgagga 300
   aaaccggcaa aaaacctcaa atcctcgacc tttagttttg ctgccacgtt gctccgcccc 360
   attgcagagt tctttttgcc cccaaatttt tttttacttg gtgcagtaag aatcgcgcct 420
   cagtgatttt ctcgactcgt agtccgttga tactgtgtct tgcttatcac ttgttctgct 480
   taatcttttt tgcttcctga ggaatgtctt ggtgcctgtc ggtggatggc gaaccaaaaa 540
   tgaagggttt tttttttttg aactgagaaa aatctttggg tttttggttg gattctttca 600
   tggagtcgcg accttccgta ttcttctctt tgatctcccc gcttgcggat tcataatatt 660
   cggaacttca tgttggctct gcttaatctg tagccaaatc ttcatatctc caggatcttt 720
   tcgctctgtc ctatcggatt taggaattag gatctaactg gtgctaatac taaagggtaa 780
   tttggaacca tgccattata attttgcaaa gtttgagata tgccatcggt atctcaatga 840
   tacttactaa aacccaacaa atccatttga taaagctggt tcttttatcc ctttgaaaac 900
   attgtcagag tatattggtt caggttgatt tattttgaat cagtactcgc actctgcttc 960
   gtaaaccata gatgctttca gttgtgtaga tgaaacagct gtttttagtt atgttttgat 1020
   cttccaatgc ttttgtgtga tgttattagt gttgatttag catggctttc ctgttcagag 1080
   atagtcttgc aatgcttagt gatggctgtt gactaattat tcttgtgcaa gtgagtggtt 1140
   ttggtacgtg ttgctaagtg taacctttct ttgcagttcc tgaaat                1186
<210> 52
   <211> 71
   <212> DNA
   <213> Triticum aestivum
<400> 52
   gatcctctag aaccatcttc cacacactca agccacacta ttggagaaca cacagggaca 60
   acacaccata a                                                      71
<210> 53
   <211> 66
   <212> DNA
   <213> Artificial Sequence
<220>
   <223> synthetic
<400> 53
   gaattccctt ttcctaccta cgatccgata ccgaattttc cgagcgcaca agccaaacca 60
   aagcaa 66
<210> 54
   <211> 68
   <212> DNA
   <213> Artificial Sequence
<220>
   <223> synthetic
<400> 54
   tctagagggc caccaccacg gtgcgcgcca agacaaggca ggggagagaa attcgtcaat 60
   ccgcagca 68
<210> 55
   <211> 82
   <212> DNA
   <213> Artificial Sequence
<220>
   <223> synthetic
<400> 55
   tctagaacca ccacaccact ccaccagtaa gaagtgcagc aggtagctag taagccggcg 60
   tagctttgct cttgcagcta ga 82
<210> 56
   <211> 70
   <212> DNA
   <213> Artificial Sequence
<220>
   <223> synthetic
<400> 56
   tccacccacc cctcgatctc tcgctcgccg ccgccgatcg gatcgcgtgg ttggatcatc 60
   acaactcggc 70
<210> 57
   <211> 74
   <212> DNA
   <213> Artificial Sequence
<220>
   <223> synthetic
<400> 57
   atccatcatc tacaagagat cgatcagtag tggttagcag caactcacta tcgaacacgg 60
   tttcagctta caca 74
<210> 58
   <211> 234
   <212> DNA
   <213> Triticum aestivum
<400> 58
   aattctgcat gcgtttggac gtatgctcat tcaggttgga gccaatttgg ttgatgtgtg 60
   tgcgagttct tgcgagtctg atgagacatc tctgtattgt gtttctttcc ccagtgtttt 120
   ctgtacttgt gtaatcggct aatcgccaac agattcggcg atgaataaat gagaaataaa 180
   ttgttctgat tttgagtgca aaaaaaaagg aattagatct gtgtgtgttt tttg 234
<210> 59
   <211> 231
   <212> DNA
   <213> Triticum aestivum
<400> 59
   aattcgctcc tggccatgga gctgcttctg tctctgggtt cacaagtctc ggtgtctccg 60
   gtatcctcca atggagtctg gtctgtgtct gtcgttgcct gactgtcttt gtttctgtac 120
   catactgtga tgcagtgtta tcgtttgtat cttcaaactt ctgctggtgt ggagcagctt 180
   tggtgaacta tgaataagtg agcggagatc tgttgtgtgt tttttggatc c 231
<210> 60
   <211> 131
   <212> DNA
   <213> Triticum aestivum
<400> 60
   aattcaacaa gaacgaggga gggatacaca ggctgtttct tccaagaaat tattgtaact 60
   aatatataat gtagcccttt tcttgtgatg cggaaaatat atttgaagaa ttccaattgg 120
   attgtgaggt c 131
<210> 61
   <211> 236
   <212> DNA
   <213> Oryza sativa
<400> 61
   ctaagttggc aatgcggata aagaataact aaataaataa ataaataaat tgcaagcaat 60
   tgcgttgctg ctatgtactg taaaagtttc ttataatatc agttctgaat gctaaggaca 120
   tccctcaaga tggtctttct atttttgtgt tcccgttcca atgtactgtt cgtatcctct 180
   tggagattca tcaatatgag aaaacagaga atggacaacc ctcccttatc ttatgg 236
<210> 62
   <211> 241
   <212> DNA
   <213> Oryza sativa
<400> 62
   ttctaaatct tattattatc atcgtcgtcg tcgtctcgtc acggaattaa ttaaagtacc 60
   tactccgtac ttagctagct acaataataa ggattcattg atcactacaa gagtgatcga 120
   ctcgactgta gtatgtgtgt gcaatataat gtgctgtcta tcaacaacta ctagtattgt 180
   catttttttc gaaccaggga actttttaat gataagaaga aaaagacaag tacttattgt 240
   c                                                                 241
<210> 63
   <211> 381
   <212> DNA
   <213> Oryza sativa
<400> 63
   attcggtggc ttttgcttgg tggttctagg gcagggtttt gtgtgcttgg tgtttccgtc 60
   ttacattatc accgtattac cgcctcgtac gccaccgccg gttcctatgt cttcgctttg 120
   ttttttcgtc tgtgctatgg gaaccttttt gggtactgta ttacttgatg ctggtctgcg 180
   attgttgata tttcgggatg aattttacct ttccgcgttg gtcctcgtgt gtaatatttg 240
   caaattacgg aactaggaag gtagcccgcg cattcgcgtg ggcatgtatc gtaggctgta 300
   tttgagataa tcgtaagtaa taggctgatt gtgttaaaat gttgcatttg ttatatagta 360
   aactataggc atatggatct t                                           381

## Description

### FIELD OF THE INVENTION

The present invention relates generally to plant genetic engineering. More particularly, it concerns improved gene expression systems for transgenic plants using different combinations of genetic elements in a plant expression cassette. The present invention also relates to recombinant DNA molecules containing the genetic elements, and to microorganisms, plant cells and plants transformed with the DNA molecules.

### BACKGROUND OF THE INVENTION

Recent advances in genetic engineering have provided the requisite tools to transform plants to contain foreign genes. By constructing a desired recombinant plant gene and introducing it into plant cells it is now possible to generate transgenic plants which have unique characteristics of agronomic importance.

Notably WO95/06742 discloses plant expression constructs having DNA molecules comprising (a) a promoter, (b) a 5' non-translated leader sequence (i.a. hsp17 from soy), (c) a coding sequence and (d) a 3' non-translated terminator.

Consistent and reliable genetic elements for use in constructing recombinant plant genes are of great value in plant genetic engineering. Many such elements can enhance the levels of gene expression of a particular gene of interest. In doing so, these elements provide several advantages. First by providing improved expression levels, the optimal combinations of genetic elements can result in a more pronounced phenotype. This is due to the observed relationship in many instances between the level of transgene expression in a transgenic plant and the extent to which a desired plant characteristic is altered.

Second, non-translated genetic elements that are capable of enhancing expression can minimize some of the rate-limiting steps in transgenic plant production. The higher the levels of expression attainable, the fewer numbers of plants need to be produced and screened in order to recover those which produce quantities of a target protein or RNA molecule sufficient to result in the agronomically desired phenotype.

Finally, the identification of a variety of alternative genetic elements provides the added advantage of reducing vector element redundancies. Thus, as multiple, independent transgenes are engineered in transgenic plant lines, the use of alternative expression vector elements in the different transgenes will help minimize homology-dependent inhibition of gene expression.

### SUMMARY OF THE INVENTION

The invention disclosed herein provides novel combinations of genetic elements for use in constructing recombinant, plant-expressible DNA molecules. A recombinant DNA molecule containing the combinations of genetic elements of the present invention exhibits improved expression levels, as desired for transformation and regeneration of transgenic plants. The improved expression levels attainable using the elements of this invention provide numerous advantages, such as a reduction in the labor-intensive screening process required for transgenic plant production and enhanced phenotypes of the plants so produced. Furthermore, the elements are useful alternatives for increasing gene stacking capabilities in transgenic plants by minimizing the repetition of sequences which has been associated with instability of transgene expression.

Therefore, in accordance with one aspect of the present invention, there is provided a recombinant DNA molecule which comprises, operably linked in the 5' to 3' direction (hereinafter shortly referred to the "recombinant DNA molecule of the invention):
(a) a promoter sequence;
(b) a 5' non-translated leader sequence comprising SEQ ID NO:53;
(c) a first intron sequence isolated from a nucleotide sequence associated with a gene selected from a rice actin gene, a rice sucrose synthase gene, a rice phenylalanine ammonia lyase gene, a maize heat shock protein gene and a rice amylase gene;
(d) a DNA coding sequence; and
(e) a 3' non-translated terminator sequence isolated from a nucleotide sequence associated with a gene selected from a wheat heat shock protein gene, a wheat ubiquitin gene, a wheat fructose-1,6-bisphosphatase gene, a rice glutelin gene, a rice lactate dehydrogenase gene, and a rice beta-tubulin gene.

In another aspect of the invention, there is provided a method for enhancing gene expression in plants and increasing genetic element diversity which comprises:
(a) transforming plant cells with a recombinant DNA molecule of the invention;
(b) selecting plant cells which have been transformed; and,
(c) regenerating said plant cells to provide a differentiated plant.

Further provided by the invention are plant cells containing the DNA molecules of the invention and the tissues, seeds and differentiated plants produced therefrom.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
Fig. 1 illustrates plasmid pMON19469
Fig. 2 illustrates plasmid pMON26052
Fig 3 illustrates plasmid pMON26055
Fig. 4 illustrates plasmid pMON26054
Fig. 5 illustrates plasmid pMON 19433
Fig 6 illustrates plasmid pMON32502
Fig. 7 illustrates plasmid pMON32506
Fig. 8 illustrates plasmid pMON32509
Fig 9 illustrates plasmid pMON325 10
Fig. 10 illustrates plasmid pMON32513
Fig. 11 illustrates plasmid pMON19437
Fig 12 illustrates plasmid pMON32515
Fig. 13 illustrates plasmid pMON32516
Fig. 14 illustrates plasmid pMON32517
Fig. 15 illustrates plasmid pMON33216
Fig 16 illustrates plasmid pMON33210
Fig. 17 illustrates plasmid pMON33220

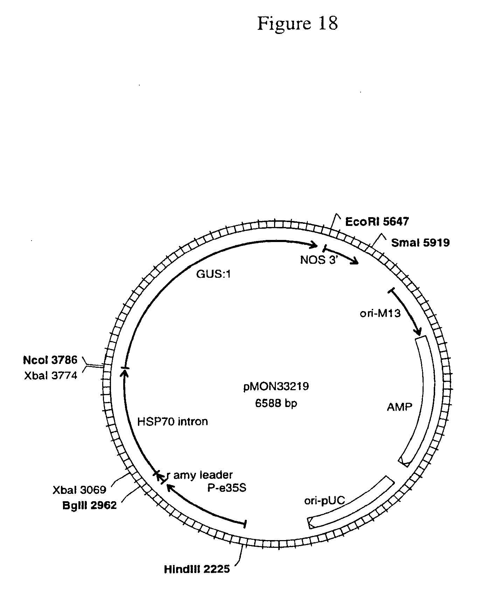

Fig. 18 illustrates plasmid pMON33219
Fig. 19 illustrates plasmid pMON47901
Fig. 20 illustrates plasmid pMON47906
Fig 21 illustrates plasmid pMON47907
Fig. 22 illustrates plasmid pMON47915
Fig. 23 illustrates plasmid pMON47916
Fig. 24 illustrates plasmid pMON47917
Fig. 25 illustrates plasmid pMON47919
Fig. 26 illustrates plasmid pMON32648
Fig. 27 illustrates plasmid pMON 18364
Fig. 28 illustrates plasmid pMON19568

### DETAILED DESCRIPTION OF THE INVENTION

The present invention provides genetic elements for improved expression of recombinant plant genes comprising novel combinations of introns, and 5' and 3' non-translated genetic elements disclosed herein. The DNA sequences and methods of the invention allow for the production of transgenic plants having increased levels of a desired RNA or protein molecule of interest, thereby facilitating the introduction of agronomically desirable traits into plants via genetic engineering.

"Recombinant plant gene" or "recombinant DNA molecule", as used in the context of this invention, refers to a combination of genetic elements that are operably linked so as to be capable of expressing in a plant cell a desired RNA and/or protein molecule. The DNA molecules can be constructed using standard techniques well known to individuals skilled in this art.

In general, a recombinant plant gene comprises, operably linked from the 5' to the 3' end: (1) a promoter region that causes the production of an RNA molecule; (2) a 5' non-translated sequence; (3) a DNA coding sequence which encodes a desired RNA and/or protein; and (4) a 3' non-translated region.

The region of a gene referred to as the "promoter" is responsible for regulating transcription of DNA into RNA. Promoters comprise the DNA sequence, usually found upstream (5') to a coding sequence, that regulates expression of the downstream coding sequence by controlling production of messenger RNA (mRNA) by providing the recognition site for RNA polymerase and/or other factors necessary for initiating transcription at the correct site. The promoter used in a recombinant plant gene of the invention is selected so as to provide sufficient transcriptional activity to achieve desired expression levels of the gene or gene(s) of interest.

Numerous plant-functional promoters are known in the art and may be obtained from a variety of sources such as plants or plant viruses and may include, the cauliflower mosaic virus (CaMV) 35S promoter (Odell et al. 1985), the Figwort mosaic virus (FMV) 35S (Sanger et al. 1990), the sugarcane bacilliform virus promoter (Bouhida et al., 1993), the commelina yellow mottle virus promoter (Medberry and Olszewski 1993), the light-inducible promoter from the small subunit of the ribulose-1,5-bis-phosphate carboxylase (ssRUBISCO) (Coruzzi et al., 1984), the rice cytosolic triosephosphate isomerase (TPI) promoter (Xu et al. 1994), the adenine phosphoribosyltransferase (APRT) promoter of *Arabidopsis* (Moffatt et al. 1994), the rice actin 1 gene promoter (Zhong et al. 1996), and the mannopine synthase and octopine synthase promoters (Ni et al. 1995). All of these promoters have been used to create various types of plant-expressible recombinant DNA constructs. Comparative analysis of constitutive promoters by the expression of reporter genes such as the uidA (β-glucuronidase) gene from E. coli has been performed with many of these and other promoters (Li et al. 1997; Wen et al. 1993). Other useful promoters include those which are expressed in a tissue-specific, tissue-enhanced, or developmentally regulated manner. Examples of these types of promoters are also known in the art.

In addition to the promoter sequence which regulates expression of operably linked DNA sequences, other genetic elements can play a role in improving gene expression. These elements include non-translated regions and intervening sequences (introns) which are associated with the genes to which they are operably linked. By "associated with" as used herein is meant that the genetic element is typically found associated with a gene during processing of the gene such as during transcription or translational processing.

5' non-translated regions of a mRNA can play an important role in translation initiation and therefore in the regulation of gene expression. A 5' non-translated leader sequence is characterized as that portion of the mRNA molecule which most typically extends from the 5' CAP site to the AUG protein translation initiation codon. For most eukaryotic mRNAs, translation initiates with the binding of the CAP binding protein to the mRNA cap. This is then followed by the binding of several other translation factors, as well as the 43S ribosome pre-initiation complex. This complex travels down the mRNA molecule while scanning for an AUG initiation codon in an appropriate sequence context. Once this has been found and with the addition of the 60S ribosomal subunit, the complete 80S initiation complex initiates protein translation (Pain, 1986; Moldave, 1985; Kozak, 1986). A second class of mRNAs have been identified which possess distinct translation initiation features. Translation from these mRNAs initiates in a CAP-independent manner and is believed to initiate with the ribosome binding to internal portions of the 5' non-translated leader sequence (Sonenberg, 1990; Carrington and Freed, 1990; Jackson *et al.,* 1990).

The efficiency of translation initiation can be influenced by features of the 5' non-translated leader sequence, therefore, identification and optimization of 5' leader sequences can provide enhanced levels of gene expression in transgenic plants. For example, some studies have investigated the use of plant virus 5' non-translated leader sequences for their effects on plant gene expression (Gallie *et al.,* 1987; Jobling and Gehrke, 1987; Skuzeski *et al.,* 1990). Increases in gene expression have been reported using the Tobacco Mosaic Virus Omega (TMV) leader sequence. When compared with other viral leader sequences, such as the Alfalfa Mosaic Virus RNA 4 (AMV) leader, two to three fold improvements in the levels of gene expression were observed using the TMV Omega leader sequence (Gallie et al., 1987; Skuzeski et al., 1990). Non-translated 5' leader sequences associated with heat shock protein genes have also been demonstrated to significantly enhance gene expression in plants (see, for example U. S. Patent 5,362,865).

Most 5' non-translated sequences are very A-U rich and are predicted to lack significant secondary structure. One of the early steps in translation initiation is the relaxing or unwinding of the secondary mRNA structure (Sonenberg, 1990). Messenger RNA leader sequences with negligible secondary mRNA structure may not require this additional unwinding step and may therefore be more accessible to the translation initiation components. Introducing sequences which can form stable secondary structures can reduce the level of gene expression (Kozak, 1988; Pelletier and Sonenberg, 1985). The ability of a 5' non-translated leader sequence to interact with translational components may play a key role in affecting the levels of subsequent gene expression.

The 5' non-translated regions which are employed in this invention are capable of increasing the level of expression of a transcribable sequence to which they are operably linked. The 5' non-translated region may be associated with a gene from a source that is native or that is heterologous with respect to the other non-translated and/or translated elements present on the recombinant gene.

The 5' non-translated sequences provided by this invention is the wheat heat shock 5' non-translated sequence (5' Ta hsp leader) of SEQ ID NO:53.

Intervening sequences herein referred to as introns are also capable of increasing gene expression. Introns can improve the efficiency of mRNA processing. A number of introns have been reported to increase gene expression, particularly in monocots. In one report, the presence of the catalase intron 1 (Tanaka, 1990) isolated from castor beans resulted in an increase in gene expression in rice but not in tobacco when using GUS as a marker gene. Still further improvements have been achieved, especially in monocot plants, by gene constructs which have introns in the 5' non-translated leader positioned between the promoter and the structural coding sequence. For example, Callis *et al.,* (1987) reported that the presence of alcohol dehydrogenase (Adh-1) introns or Bronze-1 introns resulted in higher levels of expression. Mascarenkas *et al.,* (1990) reported a 12-fold enhancement of CAT expression by use of the Adh intron. Other introns suitable for use in the DNA molecules of the invention include, but are not limited to, the sucrose synthase intron (Vasil et al., 1989), the TMV omega intron (Gallie *et al.,* 1989), the maize hsp70 intron as shown in SEQ ID NO: 47 (U.S. Patent No. 5,593,874 and U. S. Patent No. 5,859,347), and the rice actin intron (McElroy et al., 1990). A number of factors can influence the degree of enhancement of gene expression by an intron including but not limited to the promoter (Jefferson et al., 1987), flanking exon sequences and placement or location of the intron in relationship to the gene (Mascerenhas et al., 1990).

The intervening sequences provided by the present invention are associated with a plant gene, preferably monocot plant genes including wheat and rice. Particularly preferred are intervening sequences associated with monocot genes encoding heat shock proteins, actins, amylases, lyases, and synthases. These preferred intervening sequences are exemplified herein by the intervening sequence from a maize heat shock protein comprising SEQ ID NO:47, the intervening sequence from a rice actin gene comprising SEQ ID NO:50, the intervening sequence from a rice amylase gene comprising SEQ ID NO:49, the intervening sequence from a rice phenylalanine ammonia lyase gene comprising SEQ ID NO:48, and the intervening sequence from a rice sucrose synthase gene comprising SEQ ID NO:51.

Non-translated sequences located in 3' end of a gene can also influence expression levels. A 3' non-translated region comprises a region of the mRNA generally beginning with the translation termination codon and extending at least beyond the polyadenylation site. Ingelbrecht et al. (Plant Cell 1: 671-80, 1989) evaluated the importance of these elements and found large differences in expression in stable plants depending on the source of the 3' non-translated region. Using 3' non-translated regions associated with octopine synthase, 2S seed protein from Arabidopsis, small subunit of rbcS from Arabidopsis, extensin from carrot, and chalcone synthase from Antirrhinium, a 60-fold difference was observed between the best-expressing construct (which contained the rbcS 3' non-translated region) and the lowest -expressing construct (which contained the chalcone synthase 3' region). The 3' non-translated region of the nopaline synthase gene of the T-DNA in *Agrobacterium tumefaciens* (3' nos) comprising SEQ ID NO:46 has also been used as a terminator region for expression of genes in plants. While it is clear that 3' non-translated regions can significantly affect expression of recombinant plant genes, their precise role, and how to best identify and optimize them for maximal expression is an area that is not well understood.

The DNA coding sequence of a recombinant DNA molecule of the invention can encode any transcribable nucleic acid sequence including but not limited to those encoding native, foreign, and/or modified proteins of interest. Selection of this sequence will be dependent upon the objectives for a given application. Typically, the structural DNA sequence encodes a protein molecule capable of modifying one or more plant characteristics. Suitable structural genes can include, but are not limited to, genes for controlling insects and other pests, genes for controlling microbial and fungal diseases, genes for herbicide tolerance, and genes for plant quality improvements, such as yield increases, environmental tolerances, and nutritional enhancement. The genes can be isolated from any source including plants and bacteria.

Alternatively, the DNA coding sequence can effect these phenotypes by encoding a non-translatable RNA molecule that causes the targeted inhibition of expression of an endogenous gene, for example via antisense- or cosuppression-mediated mechanisms (see for example, Schuch, 1991; Bird, 1991; Jorgensen, 1990). The RNA could also be a catalytic RNA molecule (i.e.. a ribozyme) engineered to cleave a desired endogenous mRNA product (see for example, Gibson. 1997).

The 3' non-translated region which is employed in a DNA molecule described herein generally causes the polyadenylation of the 3' end of the transcribed mRNA sequence and the termination of transcription. The 3' non-translated region may be associated with a gene from a source that is native or that is heterologous with respect to the other non-translated and/or tranlsated elements present on the DNA molecule.

The 3' non-translated sequences provided by the present invention are associated with a plant gene, preferably monocot plant genes including wheat and rice. Particularly preferred are 3' non-translated sequences isolated from a nucleotide sequence associated with monocot genes encoding, a wheat fructose-1.6-bisphosphatase (Ta fbp 3') comprising SEQ ID NO:60, a wheat heat shock protein (Ta hsp 3') comprising SEQ ID NO:58, wheat ubiquitin (Ta ubiq 3') comprising SEQ ID NO:59, a rice glutelin protein (r glut 3') comprising SEQ ID NO:61, a rice lactate dehydrogenase (r lacd 3') comprising SEQ ID NO:62, and a rice beta-tubulin (r btub 3') comprising SEQ ID NO:63).

The 5' and/or 3' non-translated sequences and intervening sequences of this invention may be isolated by one or more of the numerous methods known to those of skill in the art or, alternatively, may be generated synthetically.

In one embodiment, the source plant material is plant RNA isolated from plant tissue. In another embodiment, the source material is a synthetic DNA sequence. Template sequences for the genetic elements include RNA transcripts. cDNA sequences, or genomic DNA. In another embodiment, PCR primers are synthesized to generate the genetic elements of the present invention. PCR primers can be synthesized to correspond to either the termini of 5' non-translated or 3' non-translated regions of the target plant transcripts. For example, PCR reactions on first strand cDNA products generated by reverse transcription of RNA and PCR fragment containing the desired portion or entire genetic element can be cloned into an expression vector for testing.

Methods for isolation of genes and associated genetic elements are known to those of skill in the art and would include, for example the PCR methods disclosed herein. A variety of amplification methods are known in the art and are described in for example, U.S. Patent Nos. 4,683,195 and 4,683,202 and. Innis et al.,1990. Those of skill in the art are familiar with the standard resource materials which describe specific conditions and procedures for the construction, manipulation and isolation of macromolecules (e.g., DNA molecules, plasmids, etc.), generation of recombinant organisms and the screening and isolation of genes. (see for example Sambrook et al., 1989: Mailga et al., 1995; Birren et al., 1996).

Plant molecular methods have been also been described in, e.g., Pouwels et al., 1985, supp. 1987; Weissbach and Weissbach, 1989; and Gelvin et al., 1990.

Furthermore, one skilled in the art will recognize that the 5' and/or 3' non-translated regions or intervening sequences of the invention can be modified, such as by base addition, deletion, and substitution,
while still providing the benefits disclosed herein. Such modifications are considered within the scope of this invention.

One type of modification, for example, could involve changes in the nucleotide sequence of the leader which lead to a change in secondary structure. Appropriate secondary structure of the 5' leader sequence may be required for optimal expression. As such, the specific nucleotide sequence of the leader can be important insofar as the secondary structure is concerned. Therefore, the leader sequence may in fact tolerate modifications in the nucleotide sequence which do not result in changes in secondary structure. Similarly, the introns and 3' non-translated sequences of the present invention can be modified accordingly to improve gene expression in a particular system.

Sequences surrounding the AUG of a 5' non-translated region can also affect translational efficiency. For example, a consensus sequence has been identified in plants which may provide an optimal AUG context (Joshi et al., 1987; Koziel et al., 1996). Thus, this region of the 5' non-translated sequences of the invention may be so modified to further optimize transgene expression levels. In addition, modifications can be made to other genetic components including but not limited to the 3' non-translated region or intervening sequences of the recombinant DNA molecule of the invention such that the novel combinations of elements in the expression vector are further optimized.

In addition to those elements discussed above, a recombinant DNA molecule of the invention can also include other regulatory elements such as chloroplast sequestering/targeting sequences, and enhancer elements (for review on optimizing transgene expression, see Koziel et al., 1996) For example, improvements in expression have been obtained by using enhancer sequences inserted 5' to the promoter.

A recombinant DNA molecule of the invention can also include a selectable marker. These markers are commonly used to select transformed plants or plant cells that contain the exogenous genetic material of interest, i.e., the transgene. Examples of such include, but are not limited to, a neomycin (neo) phosphotransferase gene (Potrykus *et al.,* 1985), which confers kanamycin resistance. Cells expressing the neomycin phosphotransferase gene can be selected using an appropriate antibiotic such as kanamycin or G418. Other commonly used selectable markers include the *bar* gene which confers bialaphos resistance; a mutant EPSP synthase gene (Hinchee *et al.,* 1988), which confers glyphosate resistance; a nitrilase gene which confers resistance to bromoxynil (Stalker *et al.,* 1988); a mutant acetolactate synthase gene (ALS) which confers imidazolinone or sulphonylurea resistance (European Patent Application 154,204, 1985); and a methotrexate resistant DHFR gene (Thillet *et al.,* 1988).

A recombinant DNA molecule of the invention can also include a screenable marker as an additional means by which gene expression can be evaluated. Common screenable markers include a β-glucuronidase or uidA gene (GUS) which encodes an enzyme for which various chromogenic substrates are known (Jefferson, 1987); Jefferson *et al*., 1987); a luciferase gene (Ow *et al.,* 1986), an R-locus gene which encodes a product that regulates the production of anthocyanin pigments (red color) in plant tissues ((Dellaporta 1988); a β-lactamase gene (Sutcliffe *et al.,* 1978) which encodes an enzyme for which various chromogenic substrates are known (e.g., PADAC, a chromogenic cephalosporin); a xylE gene (Zukowsky *et al.,* 1983) which encodes a catechol dioxygenase that can convert chromogenic catechols; an α-amylase gene (Ikatu *et al.,* 1990); a tyrosinase gene (Katz *et al.,* 1983) which encodes an enzyme capable of oxidizing tyrosine to DOPA and dopaquinone which in turn condenses to melanin; an α-galactosidase gene which encodes an enzyme whose substrate is chromogenic α-galactose; etc.

The terms "selectable" and "screenable" are also intended to encompass genes which encode "scriptable" markers whose secretion can be detected as a means of identifying or selecting for transformed cells. Examples include markers which encode a secretable antigen that can be identified by antibody interaction, or even secretable enzymes which can be detected catalytically. Secretable proteins fall into a number of classes, including small, diffusible proteins detectable, *e.g*., by ELISA, small active enzymes detectable in extracellular solution (*e.g.,* α-amylase, β-lactamase, phosphinothricin transferase), or proteins which are inserted or trapped in the cell wall (such as proteins which include a leader sequence such as that found in the expression unit of extensin or tobacco PR-S). Other possible selectable/screenable/scriptable marker genes will be apparent to those of skill in the art.

It is understood that the particular nucleotide sequences of the 5' and 3' non-translated elements disclosed herein are representative in the sense that equivalent sequences or portions thereof may be obtained and/or generated pursuant to this disclosure. By equivalent it is meant that said gene or portion thereof would function in a manner substantially the same as the element or portion thereof disclosed herein, and would provide a benefit or particular characteristic to a plant in substantially the same manner.

A wide variety of cloning methods and tools are commercially available and have been extensively described (see for example. Sambrook *et al.,* 1989; Birren *et al.,* 1996). Such methods are well known and can be readily used by those of skill in the art in constructing the DNA molecules of this invention.

Any type of vector can be used in the present invention, including but not limited to an *E*. *coli* plasmid expression vector. More preferably, the combinations of genetic elements of the present invention are operably linked in a plant transformation vector. In constructing a recombinant DNA molecule of the invention, the various components or fragments thereof are typically inserted using methods known to those of skill in the art into a convenient cloning vector which is capable of replication in a bacterial host, such as *E*. *coli.* Numerous vectors exist that have been described in the literature. After each subcloning, the vector may be isolated and subjected to further manipulation, such as restriction digestion, insertion of new fragments, ligation, deletion, resection, insertion, *in vitro* mutagenesis, addition of polylinker fragments, in order to provide a vector which will meet a particular need. Once the construct is completed, the construct can be be transferred to an appropriate vector for further manipulation in accordance with the manner of transformation of the plant cell. A number of plant transformation vectors have been described, and the particular vector may be modified depending on the transformation method. In one embodiment, a plant transformation vector suitable for *Agrobacterium*-mediated plant transformation can be used. In another embodiment, a plant transformation vector suitable for particle bombardment can be used. A typical plant expression vector for *Agrobacterium*-mediated plant transformation, for example, can include a number of genetic components, including but not limited to a promoter, one or more genes of interest, and a terminator sequence.

By genetic component as used herein is meant any nucleic acid sequence or genetic element which may also be a component or part of a vector. The plant expression vector also can contain the functions for mobilization from *E. coli* to *Agrobacterium* and for replication of the vector in these hosts (*i.e. E. coli* and broad host range origin of replication). In addition one or more selectable marker gene(s) for selection of bacterial cells containing the vector and for selecting plant cells containing the introduced DNA can be components of the plant expression vector. The vector also typically can contain one or more T-DNA borders which function to transfer the DNA to the plant cell. A number of vectors suitable for stable transformation of plant cells or for the establishment of transgenic plants have been described in, e.g., Pouwels et al., Cloning Vectors: A Laboratory Manual, 1985, supp. 1987); Weissbach and Weissbach, Methods for Plant Molecular Biology, Academic Press, 1989; Gelvin et al., Plant Molecular Biology Manual. Kluwer Academic Publishers, 1990; and R.R.D. Croy Plant Molecular Biology LabFax, BIOS Scientific Publishers, 1993. The optimal plant transformation vector can be designed for the particular DNA delivery method and target crop of interest.

Bacterial or viral cells comprising DNA molecules containing the non-translated sequences of the present invention are also encompassed by the present invention. The introduction of such vectors into a host may be accomplished using methods known to those of skill in the art.

A DNA molecule of the present invention can be inserted into the genome of a plant by any suitable method. Suitable plant transformation methods include *Agrobacterium*-mediated transformation, the use of liposomes, electroporation, chemicals that increase free DNA uptake, free DNA delivery via microprojectile bombardment, transformation using viruses or pollen.

Methods for specifically transforming dicots primarily use *Agrobacterium tumefaciens.* For example, transgenic plants reported include cotton (U. S. Patent No. 5,004,863: U. S. Patent No. 5,159,135; U. S. Patent No. 5,518,908, WO 97/43430) and soybean (U. S. Patent No. 5,569,834; U. S. Patent No. 5,416,011).

Similarly a number of transformation and regeneration methods are available for monocots including corn (Songstad et al.1995; Klein et al., 1988); rice (Toriyama et al.1986); and wheat (Cheng et al.1997; and U. S. Patent No. 5,631,152). It is apparent to those of skill in the art that a number of transformation and regeneration methodologies can be used and modified for production of stable transgenic plants from any number of target crops of interest and methods of plant transformation and regeneration are well known to the skilled individual (for example, see Hinchee et al. (1994), and Ritchie & Hodges (1993) for reviews).

Assays for gene expression based on the transient expression of cloned nucleic acid constructs have been developed by introducing the nucleic acid molecules into plant cells by polyethylene glycol treatment, electroporation, or particle bombardment (Marcotte, *et al.,* (1988); Marcotte, *et al.,* (1989),; McCarty, *et al.,* (1991); Hattori, *et al.,* (1992); Goff, *et al*., (1990). Transient expression systems can be used to quickly assess gene expression levels and functionally dissect gene constructs (See *generally,* Mailga *et al..* (1995)).

The present invention also provides plant cells, the genome of which contains one or more recombinant DNA molecules comprising a 5' and/or 3' non-translated sequence described herein. Differentiated plants comprising such cells will have the features or benefits provided by the expression of the DNA coding sequence that is operably linked to said sequences. Such plants may be monocots or dicots, and may include plants belonging to families selected from alfalfa, apple, Arabidopsis, barley, Brassica, broccoli, cabbage, citrus, corn, cotton, flax, garlic, lettuce, oat, oilseed rape, onion, canola, flax, an ornamental plant, pea, peanut, pepper, potato, rice, rye, sorghum, strawberry, soybean, sunflower, sugarcane, sugarbeet, tomato, tobacco, wheat, poplar, pine, fir, eukalyptus, lentil, grape, banana, tea, turf grasses. Particularly preferred plants include alfalfa, barley, corn, cotton, canola, potato, rice, rye, soybean, sunflower, sugarbeet, and wheat. Even more preferred plants include monocots such as corn, wheat, and rice.

The invention will be more readily understood through reference to the following examples. Examples not falling within the scope of the claims are for illustrative purposes.

In addition to the procedures specifically referenced herein, practitioners are familiar with the standard resource materials which describe conditions and procedures for the construction, manipulation and isolation of macromolecules (*e.g*., DNA molecules, plasmids), for the generation of recombinant organisms and for the screening and isolation of clones, (*see* for example, Sambrook *et al.,* (1989); Mailga *et al..* (1995); Birren *et al.,* (1996).

### EXAMPLES

### EXAMPLE 1

### Construction of Expression Vectors Containing Combinations of Genetic Elements for Improved Transgene Expression

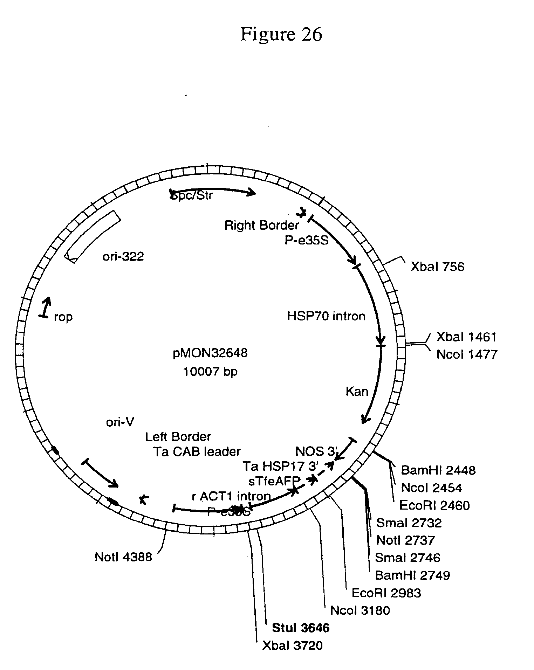

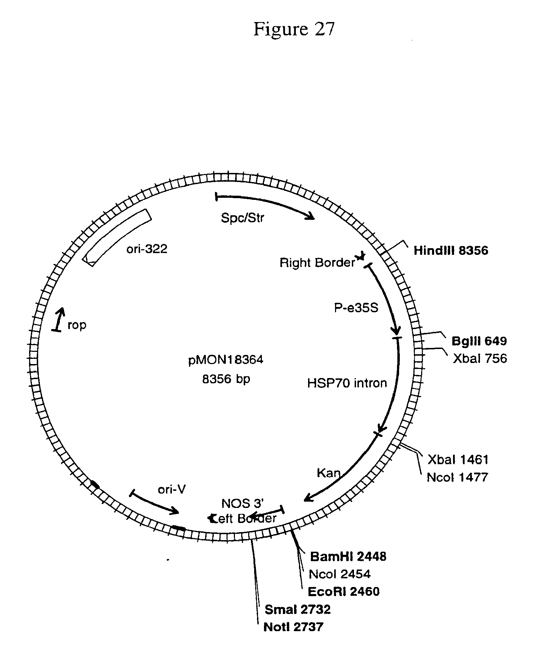

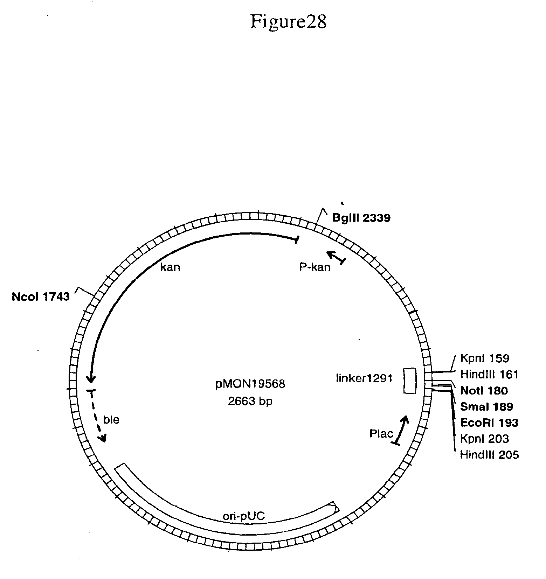

Construction of the expression vectors containing the genetic element cassettes comprising wheat or rice elements was performed by annealing synthetic oligonucleotides or by the PCR isolation from wheat leaf mRNA or rice genomic DNA and ligation into restriction sites upstream and downstream, respectively, of the GUS reporter gene. The plasmids used for cloning and construction of the various expression cassettes are listed in Table 1. All constructs tested had the e35S promoter which is the promoter for 35S RNA from CaMV containing a duplication of the -90 to 300 region. Other elements contained on the plasm ids include the following: origins of replication (ori-M 13 and ori-V), marker genes such as GUS or LUC which are the coding sequences for beta-glucuronidase and luciferase, respectively, and coding sequences for antibiotic selection (AMP bacterial selection), and KAN (confers resistance to neomycin and kanamycin aminoglycoside antibiotics). Plasmid pMON32648 (Fig. 26) contains an antifungal protein from tall fescue (Tfe AFP) as described in patent application 60/097150. Additional typical transformation vectors would include but are not limited to pMON 18364 (Fig. 27) which is a double border Agrobacterium transformation vector and pMON19568 (Fig. 28) which is a plasmid that is linearized prior to a particle bombardment transformation method. PCR conditions used were as recommended by the manufacturer (see for example, Strategene, La Jolla, CA, PE Biosystems, Foster City, CA). Plasmid DNA was isolated and purified using commercially available kits (see for example Qiagen, Valencia, CA). Synthetic DNA was purchased from Midland Certified Reagent Co., Midland, TX).

**TABLE 1. Construction of Vectors ***

| Construct | (5' Leader / Intron / Marker /3' Terminator)** | Cloning Sites/ (genetic element) |
|---|---|---|
| pMON19469* | none / hsp70 I / GUS / nos 3' (Fig. 1) | BglII, NcoI (hsp70 I) |
| pMON26043 | none / GUS / nos 3' | BglII, NcoI, XbaI |
| pMON26052* | Ta hsp L / hsp70 I / GUS / nos 3' (Fig. 2) | BglII, Ncol (hsp70 I) |
| pMON25454 | rice actin intron vector | Stu I / NcoI (ractl I) |
| pMON26044 | Ta cab L/ ractl I / GUS / nos 3' | NcoI / Stu (ractl I), EcoRI/Sma (nos3') |
| pMON26055* | Ta hsp L/ ractl I / GUS / nos 3' (Fig. 3) | Stu I/ NcoI (ractl 1) |
| pMON26045 | Ta fbp L/ GUS / nos 3' | HindIII, Xba, BgIII, Ncol |
| pMON25456 | none / ractl I / GUS / nos 3' | HindIII / BcII (e35S/ractl I) |
| pMON26064 | ractI / Ta fbp L / GUS / nos 3'** | HindIII, Xba, BcII |
| pMON26054* | Ta cab L/ ractl I / GUS / nos 3' | StuI/NcoI (ractl I), see Fig.4 EcoRI/SmaI (nos 3') |
| pMON26038 | Ta cab L/ GUS / nos 3' | XbaI, BgIII, Ncol, PstI, (Pst/BgIII, nos 3') |
| pMON19433* | none / hsp70 I / GUS / nos 3' | EcoRI/Bam HI (nos 3') BglII/EcoRL, see Fig. 5 |
| pMON18375 | none / hsp70 I / GUS / Ta hsp17 3' | EcoRI / SmaI (Ta hsp17 3') |
| pMON32502* | Ta cab L/ ractl I/ GUS/ Ta hsp17 3' | see Fig. 6 |
| pMON32506* | Ta hsp L / ractl I / GUS / Ta hsp17 3' | see Fig. 7 |
| pMON18377 | none / hsp70 I / GUS / Ta ubiq 3' | EcoRI / Sma I (Ta ubiq 3') |
| pMON32509* | Ta fbp L / ractl I / GUS / Ta ubiq 3' | see Fig. 8 |
| pMON32510* | Ta hsp L/ ractl I / GUS / Ta ubiq 3' | see Fig. 9 |
| pMON18379 | none / hsp70 I / GUS / Ta fbp 3' | |
| pMON32513* | Ta fbp L / ractl I / GUS / Ta fbp 3' | see Fig. 10 |
| pMON19437* | none / hsp70 I / LUX / nos 3' | Ncol / EcoRI (LUX) see Fig. 11 |
| pMON32515* | Ta cab L / ractl I / LUX / Ta hsp 3' | NcoI / EcoRI (LUX) see Fig. 12 |
| pMON32516* | Ta fbp L / ractl I / LUX / Ta ubiq 3' | NcoI / EcoRI (LUX) see Fig. 13 |
| pMON32517* | Ta fbp L / ractl I / LUX / /Ta fbp 3' | Ncol / EcoRI (LUX) see Fig. 14 |
| pMON32518* | Ta fbp L / ractl I / LUX / r lac d 3' | see Fig. 15 |
| pMON33210* | none/ hsp70 I/ GUS/ nos | PmlI, BglII, XbaI (r btubL) see Fig. 16 |
| pMON33220* | r btub L / hsp70 I /GUS / nos 3' | see Fig. 17 |
| pMON26046 | Ta per L / none / GUS/ nos | BglII/NcoI (Ta per L) PstI/BglII |
| pMON33211 | none/ ramyl I / GUS / nos 3' | BglII / XbaI (r amy I) |
| pMON33226 | none/ r pal I / GUS / nos 3' | BglII / Xba (r pal 1) |
| pMON33228 | none/ r ss l I / GUS / nos 3' | BglII / Xba (r ssl I) |
| pMON33225 | Ta cab L / ractl I / GUS / r glut 3' | EcoRI / Sph (r glut 3') |
| pMON33200 | none / hsp70 I / GUS / nos 3' | BglII / Pmll |
| pMON33219* | r amy L / hsp70 I / GUS/ nos | see Fig. 18 |
| pMON47901* | Ta cab L/ hsp70 I / GUS / glut 3' | see Fig. 19 |
| pMON47906* | Ta hsp L / ractl I / GUS / r lac d3' | see Fig. 20 |
| pMON47907* | Ta hsp L/ ractl I / GUS / r glut 3' | see Fig. 21 |
| pMON47915* | Ta per L / ractl I / GUS / r lac d 3' | see Fig. 22 |
| pMON47916* | Ta per L / ractl I / GUS / r glut 3' | see Fig. 23 |
| pMON47917* | Ta per L / ractl I / GUS / r btub 3' | see Fig. 24 |
| pMON47919* | | see Fig. 25 |
| pMON47909 | Ta hsp L / ract 1 I / GUS / r lac d 3' | BglII/NcoI |
| pMON33216 | Ta cab L/ ractl I/ GUS / r btub 3' | StuI/ Ncol |
| pMON47910 | Ta hsp L / ractl I / GUS / r glut 3' | NcoI / BglII (hsp70 I) |
| pMON47918 | Ta per L / hsp70 I / GUS / r lac d 3' | NcoI / BglII (hsp70 I) |
| pMON47920 | Ta per L / hsp70 I / GUS / r btub | BglII / NcoI (hsp70 I) |

| | | |
|---|---|---|
| * Figure ** Different orientation: Intron/leader/marker/3' | | |

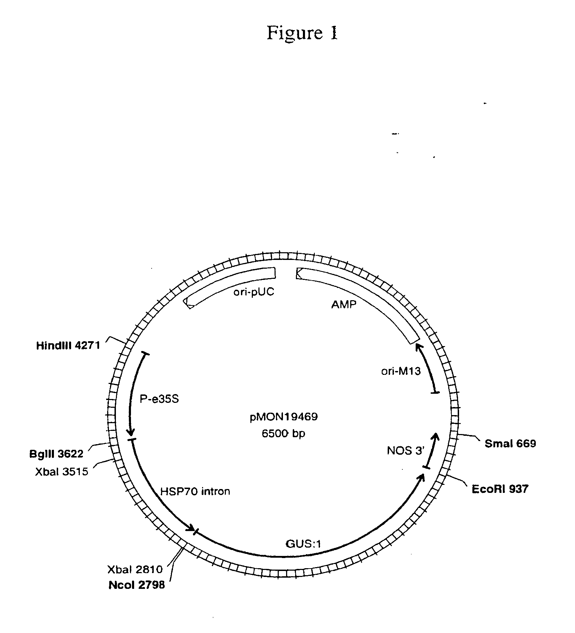

The intervening sequence from the corn heat shock protein (hsp70 intron) as described in U. S. Patent Nos. 5,593,874 and 5,859,347 herein incorporated by reference) is shown in SEQ ID NO: 47. The base synthetic leader is shown in SEQ ID NO:45. The 5' non-translated leader sequence from the wheat mRNA for putative low molecular weight heat shock protein (5' Ta hspl7 L) (Genebank Accession Number X13431.gb_pl) (SEQ ID NO:53) was created by annealing SEQ ID NO. 5, SEQ. ID NO. 6, SEQ ID NO.7, and SEQ ID NO. 8. The 5' end of the resultant fragment has a BamHI cohesive end followed by an Xbal restriction site, and the 3' end has a BglII site followed by an NcoI cohesive end for subcloning purposes. This fragment was ligated with the 5.676 kb BglII and NcoI fragment of pMON1 19469 (Figure 1) to create pMON26043.

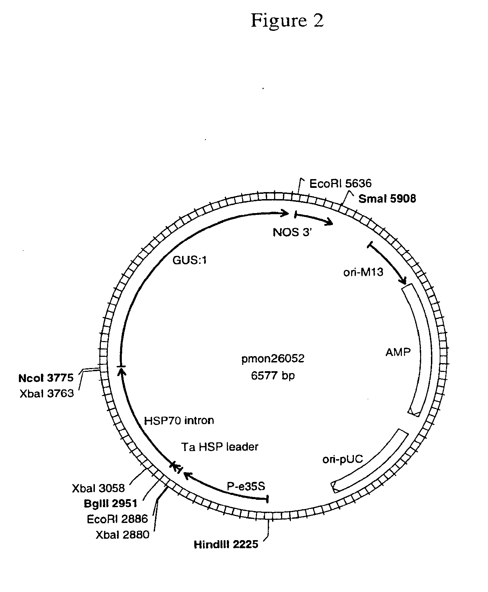

Plasmid pMON26052 (Figure 2) was created by subcloning the 884 bp BgIII and NcoI fragment from pMON 19469 (Figure 1) into the BgIII and Ncol sites of pMON26043. PMON26043 contained both the HP70 intron and 5' wheat leader sequence (Ta hsp leader).

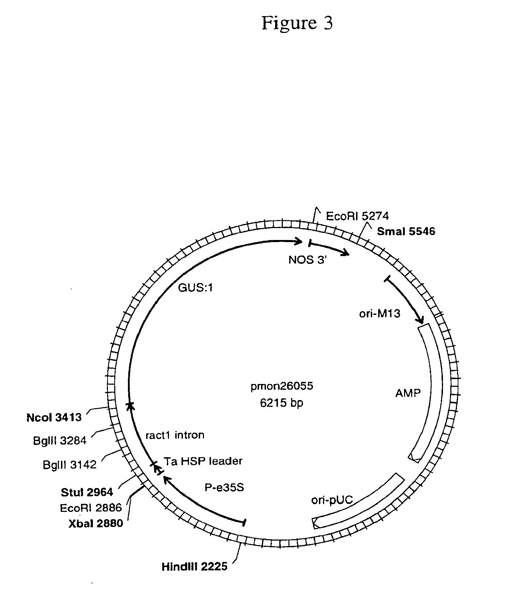

PMON26055 (Figure 3) was created by subcloning the 0.449 kb Stul and Ncol fragment containing the rice actin intron (ract1 intron) (SEQ ID NO:50) (McElroy, et al., 1991) from pMON25454 into the BglII and Ncol sites of pMON26043 using adaptors to the Stul site which create a BglII complementary end (SEQ ID NO. 3 and SEQ ID NO. 4).

The 5' non-translated leader from the wheat mRNA for fructose-1, 6-bisphosphatase (5' Ta fbp L) (Genebank Accession Number X07780.gb_pl) (SEQ ID NO:54) was created by annealing SEQ ID NO. 9, SEQ ID NO. 10, SEQ ID NO. 11, and SEQ ID NO. 12. The 5' end of the resultant fragment has a BamHI cohesive end followed by an XbaI restriction site, and the 3' end has a BglII site followed by an NcoI cohesive end for subcloning purposes. This fragment was ligated with the 5.676 kb BglII and NcoI fragment of pMON 19469 (Figure 1) to create pMON26045.

Plasmid pMON26064 was created by subcloning the 1.095 kb HindIII and BclI fragment containing the rice actin intron (McElroy, et. al., 1991) and the e35S promoter (Kay et. al., 1987) from pMON25456 into the HindIII and Xbal sites of pMON26045 using adaptors to the BclI site which create an Xbal complementary end (SEQ ID NO. 13 and SEQ ID NO. 14).

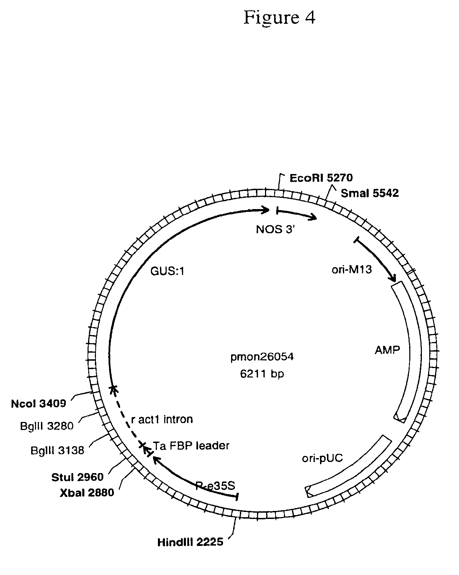

PMON26054 (Figure 4) was created by subcloning the 0.449 kb StuI and NcoI fragment containing the rice actin intron (McElroy, et. al., 1991) from pMON25454 into the BglII and NcoI sites of pMON26045 using adaptors to the Stul site which create a BglII complementary end (SEQ ID NO. 3 and SEQ ID NO. 4).

The major chlorophyll a/b binding protein 5' untranslated leader (5' Ta cab L) (Genebank Accession Number M10144.gb_pl) (SEQ ID NO:52) was isolated via reverse transcription from wheat leaf RNA followed by 40 cycles of PCR with a denaturation temperature of 94 °C for I minute, an annealing temperature of 50 °C for 2 minutes, and an extension temperature of 72 °C for 3 minutes. The primers used, SEQ ID NO. 1 and SEQ ID NO. 2, create BamHI and Xbal restriction sites at the 5' end, and BglII and Ncol restriction sites at the 3' end for subcloning purposes. The 77 base pair fragment containing the major chlorophyll a/binding protein 5' untranslated leader was then digested with BamHI and Ncol and ligated with the 5.676 kb BglII and NcoI fragment of pMON 19469 (Figure 1) to create pMON26038.

Plasmid pMON26044 was created by subcloning the 0.449 kb StuI and Ncol fragment containing the rice actin intron (McElroy. et al., 1991) from pMON25454 into the BglII and Ncol sites of pMON26038 using adaptors to the StuI site which create a BglII complementary end (SEQ ID NO. 3 and SEQ ID NO. 4).

The 3' non-translated regions containing terminator and polyadenylation sequences were isolated and cloned into pMON19433. The plasmid pMON19433 is derived from pUC119 and in addition contains the CAMV enhanced 35S promoter, the hsp70 intron, the GUS reporter gene, and the nopaline synthase (nos) 3' non-translated sequence (SEQ ID NO:46). This vector contains an EcoRI site and a BamHI site flanking the nos 3' terminator allowing removal and substitution of alternative 3' termination sequences. The synthesis of cDNA was performed using a oligo dT primer in PCR reaction buffer with MgCl₂, 0.2 mM dATP, dCTP, dGTP, and TTP. Five microgram of wheat leaf total cellular RNA was added to the PCR reaction buffer in a 20 µl volume. The reaction was initiated by the addition of 4 units reverse transcriptase (Gibco BRL; Gaithersburg, MD), and incubated at 42 °C for 2 hours. The reaction was heat terminated and frozen at -20 °C.

Two microliters of this reaction was added to each PCR reagent buffer containing dNTPs as described above in 100 microliters containing 0.5 units of Taq polymerase according to the specifications of the manufacturer (Boerhinger Mannheimm Biochemicals; Indianapolis, IN). The reaction was overlayered with 50 microliters of mineral oil and cycled in a thermocycler at 94 °C for 1 minute, 45 °C for 2 minutes, and 72 °C for 2 minutes repeatedly for 40 cycles. The 3' non-translated region from the wheat ubiquitin gene (3' Ta ubiq) (SEQ ID NO:59) was amplified from the cDNA products with primer sequences SEQ ID NO. 15 and SEQ ID NO. 16. The 3' non-translated region from the wheat heat shock gene (3' Ta hsp17) (SEQ ID NO:58) was amplified as described above using primer sequences SEQ ID NO. 17 and SEQ ID NO. 18. The 3' non-translated region from fructose bisphosphatase (3' Ta fbp) (SEQ ID NO:60) was amplified as described above using primer sequences SEQ ID NO. 19 and SEQ ID NO. 20.

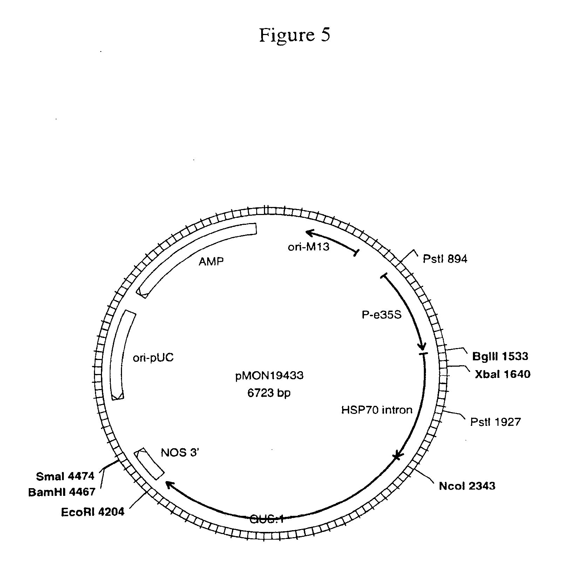

The amplified reaction products were electrophoresed on an agarose gel and analyzed for size. PCR fragments corresponding to 3' Ta ubiq (∼225 bp), 3' Ta hsp (∼240 bp) and 3' ta fbp (∼130 bp) were digested with EcoRI and BamHI, gel purified on a 1% agarose gel and isolated by using the Qiagen PCR prep according the manufacturer' s specification (Qiagen, Santa Clarita, CA). The end digested PCR fragments were cloned into the 6.5 kb fragment from EcoRI BamHI digested base vector pMON19433 (Figure 5). The resulting transformants with inserts from 3' non-translated regions of wheat genes (Ta fbp, Ta hsp, and Ta ubiq) were verified by DNA sequencing.

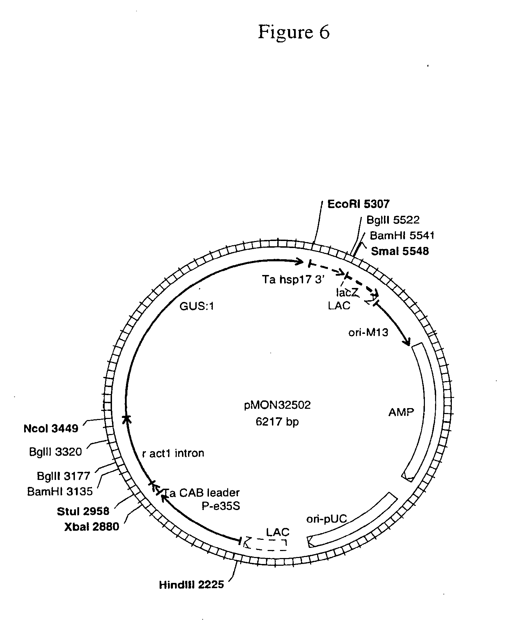

The pMON32502 (Figure 6) expression vector was constructed by digestion of pMON26044 and pMON18375 with EcoRI and SmaI. The 0.24 kb Ta hsp 3' fragment from pMON18375 was ligated with the 6.0 kb vector backbone fragment of pMON26044 creating pMON32502. The ligation products were transformed into *E. coli* DH5 alpha cells by standard procedures, plated on LB agar plates containing selective levels of ampicillin (100 µg/ml).

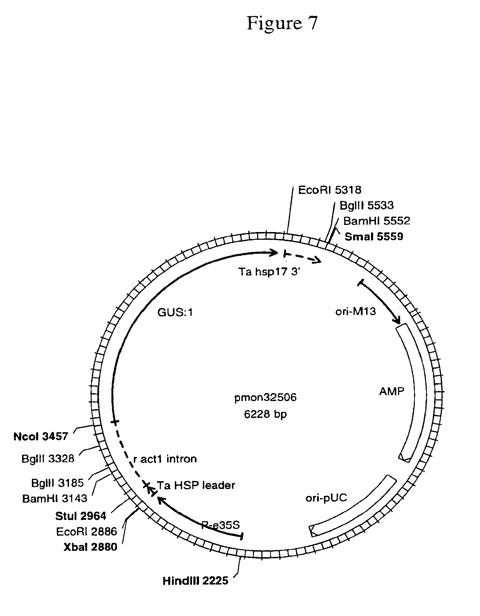

The pMON32506 (Figure 7) expression vector was constructed by digestion of pMON26055 (Figure 3) and pMON18375 with EcoRI and SmaI. The 0.24 kb Ta hsp 3' fragment from pMON18375 was ligated with the 5.9 kb vector backbone fragment of pMON26055 (Figure 3) creating pMON32506. The ligation products were transformed into *E. coli* DH5 alpha cells by standard procedures.

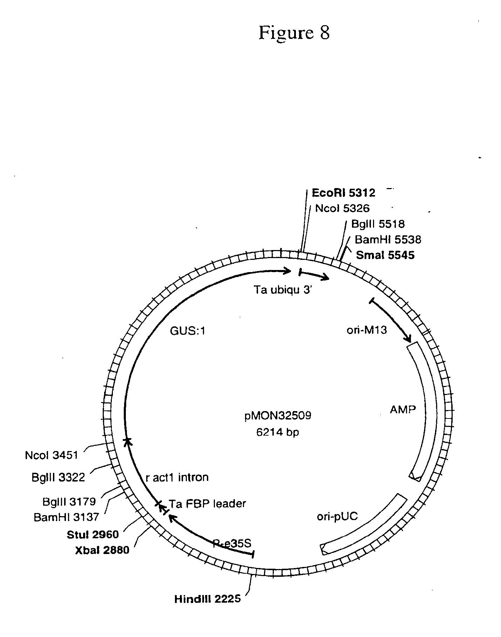

The pMON32509 (Figure 8) expression vector was constructed by digestion of pMON26054 and pMON18377 with EcoRI and SmaI. The 0.23 kb Ta ubiq 3' fragment from pMON18377 was ligated with the 5.9 kb vector backbone fragment of pMON26054 (Figure 4) creating pMON32509. The ligation products were transformed into *E. coli* DH5 alpha cells by standard procedures.

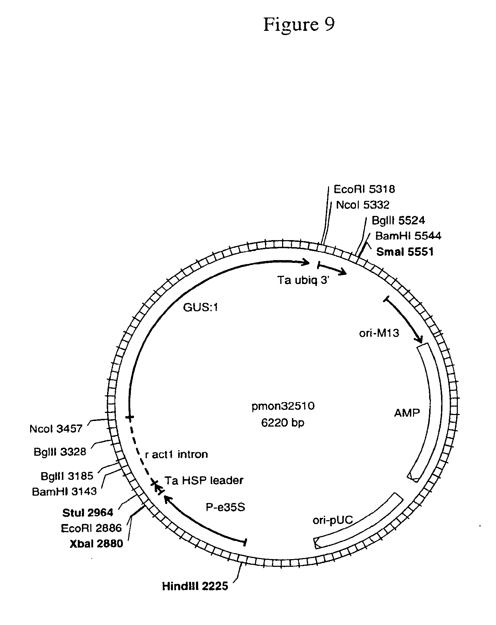

PMON32510 (Figure 9) was constructed by digestion of pMON26055 (Figure 3) and pMON18377 with EcoRI and SmaI. The 0.23 kb Ta ubiq 3' fragment from pMON18377 was ligated with the 5.9 kb vector backbone fragment of pMON26055 creating pMON32510 (Figure 9). The ligation products were transformed into *E. coli* DH5 alpha cells by standard procedures.

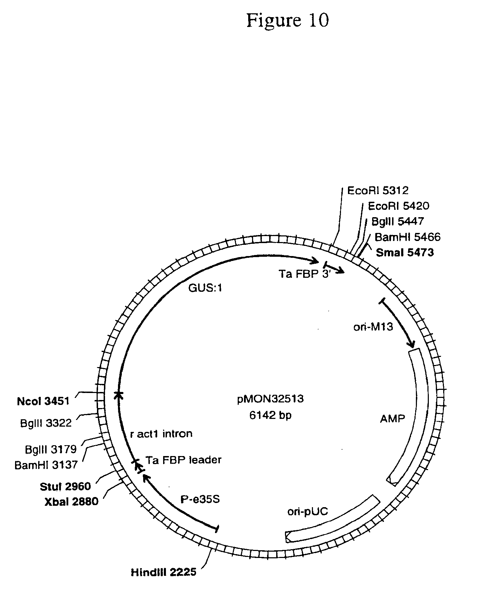

PMON32513 (Figure 10) was constructed by digestion of pMON26054 and pMON 18379 with NcoI and SmaI. The 2.0 kb GUS/Ta fbp 3' sequence fragment from pMON 18379 was ligated with the 4.1 kb vector backbone fragment of pMON26054 creating pMON32513. The ligation products were transformed into *E. coli* DH5 alpha cells by standard procedures.

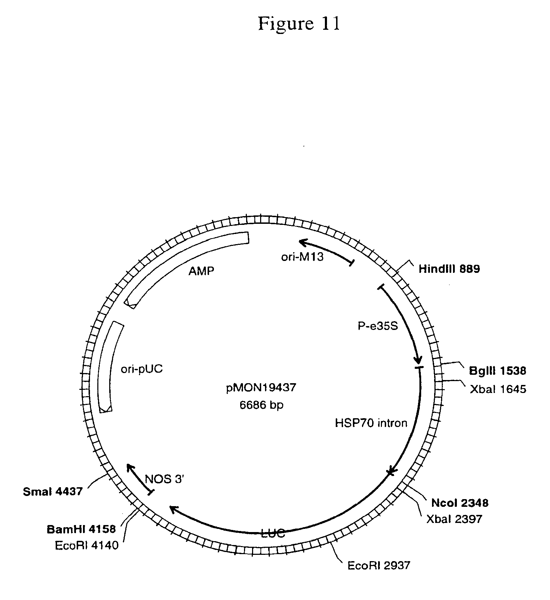

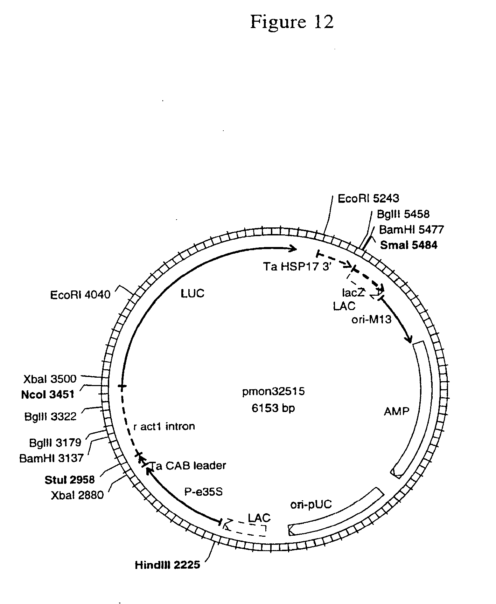

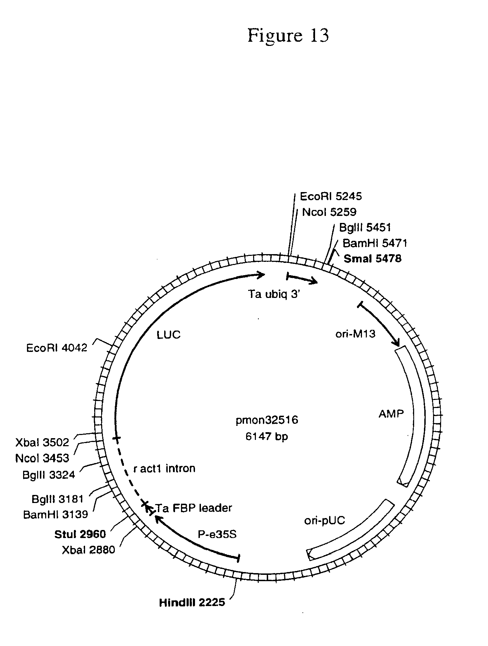

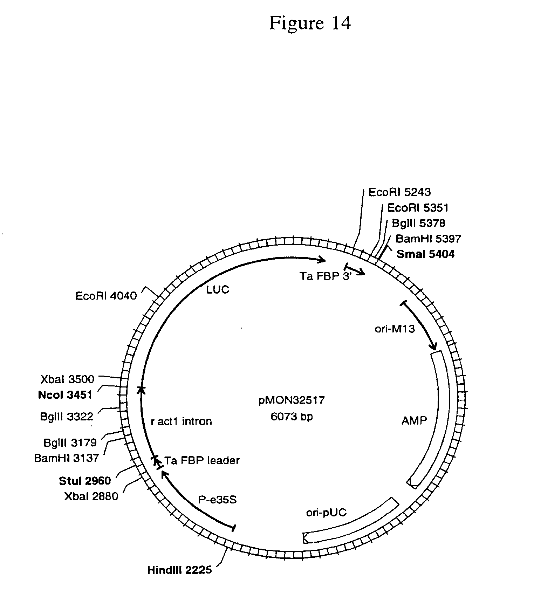

Plasmids pMON32515 (Figure 12), pMON32516 (Figure 13) and pMON32517 7 (Figure 14) were constructed by digesting pMON32502, pMON32509, and pMON32513 with NcoI and EcoRI. In each case, the approximate 4.3 kb fragment was then ligated to the 1.8 kb luciferase NcoI fragment created by a partial digestion with EcoRI from pMON19437 (Figure 11). The ligation products from these ligations were transformed into *E. coli* DH5 alpha cells by standard procedures.

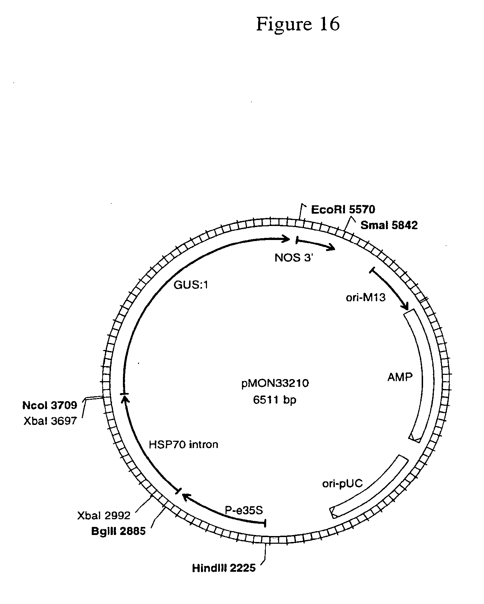

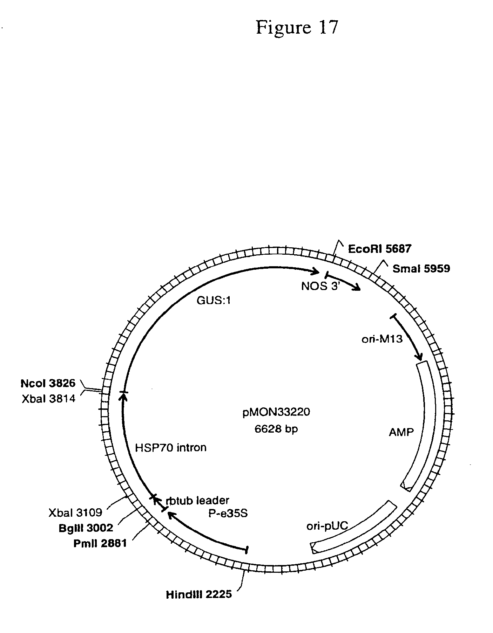

The 5' non-translated leader sequence from rice beta-tubulin (5' r btub L) (Genebank Accession Number L19598.gb_pl) (SEQ ID NO:56) was created by kinasing (reaction using T4 polynucleotide kinase for adding 5' phosphates for subsequent ligation steps) and annealing (boiling followed by slow cooling) SEQ ID NO:21, SEQ ID NO:22, SEQ ID NO:23, and SEQ ID NO:24. The 5' end of the resultant fragment has a Pml I blunt end and the 3' end has a BglII cohesive end for subcloning purposes. This fragment was ligated with the 6.507 kb Pmll and BglII fragment of pMON33210 0 (Figure 16) to create pMON33220 (Figure 17).

The 5' non-translated leader sequence from a wheat peroxidase gene (5' Ta per L) (Genebank Accession Number X56011.gb_pl) (SEQ ID NO:55) was created by kinasing and annealing SEQ ID NO:25, SEQ ID NO:26, SEQ ID NO:27, and SEQ ID NO:28. The 5' end of the resultant fragment has a BglII cohesive end followed by an XbaI restriction site, and the 3' end has a BglII site followed by an Ncol cohesive end for subcloning purposes. The fragment was ligated with the 5.676 kb BglII and NcoI fragment of pMON 19469 (Figure 1) to create pMON26046.

The 5' non-translated leader sequence from a rice amylase gene (Genebank Accession Number M24287.gb_pl (5' r amy L) (SEQ ID NO:57) was created by annealing SEQ ID NO:41, SEQ ID NO:42, SEQ ID NO:43, and SEQ ID NO:44, and phosphorylating and ligating into linearized plasmid pMON33200 digested with PmlI and BglII, to create pMON33219.

The first intron of the rice amylase gene (r amy1 intron) (Genebank Accession Number X 16509.gb_pl) (SEQ ID NO:49) along with 10 base pairs of flanking 5' and 3' exon sequence was isolated by PCR of rice (Oryza sativa) genomic DNA, using about 1 µg DNA. Amplification was performed using primer sequences SEQ ID NO:29 and SEQ ID NO:30. The DNA was denatured for 1 minute at 95 °C, annealed for 2 minutes at 50 °C, and extended for 3 minutes at 72 °C for a total of 30 cycles. The resultant PCR product was digested with BglII and XbaI and ligated with the 5.687 kb BglII and XbaI fragment of pMON33210 (Figure 16) to create pMON33211.

The rice phenylalinine ammonia lyase intron (r pal intron) (Zhu et al., 1995) along with 10 base pairs of flanking 5' and 3' exon sequence (SEQ ID NO:48) was isolated by PCR of rice genomic DNA. Amplification was performed using primer sequences SEQ ID NO:31 and SEQ ID NO:32. The DNA was denatured for 1 minute at 95 °C annealed for 2 minutes at 50 °C and extended for 3 minutes at 72 °C for a total of 30 cycles. The resultant PCR product was digested with BglII and XbaI and ligated with the 5.687 kb BglII and XbaI fragment of pMON33210 (Figure 16) to create pMON33226.

The first intron of the rice sucrose synthase gene (r ss1 intron) (Wang et al., 1992) along with 10 base pairs of flanking 5' and 3' exon sequence (SEQ ID NO:51) was isolated by PCR of rice genomic DNA. Amplification was performed using primer sequences SEQ ID NO:33 and SEQ ID NO:34. The DNA was denatured for 1 minute at 95 °C, annealed for 2 minutes at 50 °C, and extended for 3 minutes at 72 °C for a total of 30 cycles. The resultant PCR product was digested with BglII and XbaI and ligated with the 5.687 kb BglII and Xbal fragment of pMON33210 (Figure 16) to create pMON33228.

The 3' non-translated terminator from rice glutelin type II (3' r glut) (Genebank Accession Number X05664.gb_pl) (SEQ ID NO:61) was isolated by PCR of rice genomic DNA. Amplification was performed using primer sequences SEQ ID NO:35 and SEQ ID NO:36. The DNA was denatured for I minute at 95 °C, annealed for 2 minutes at 50 °C, and extended for 3 minutes at 72 °C for a total of 30 cycles. The resultant PCR product was digested with SphI and EcoRI. PMON33225 contains the cloned SphI/ EcoRI r glut 3' non-translated terminator region.

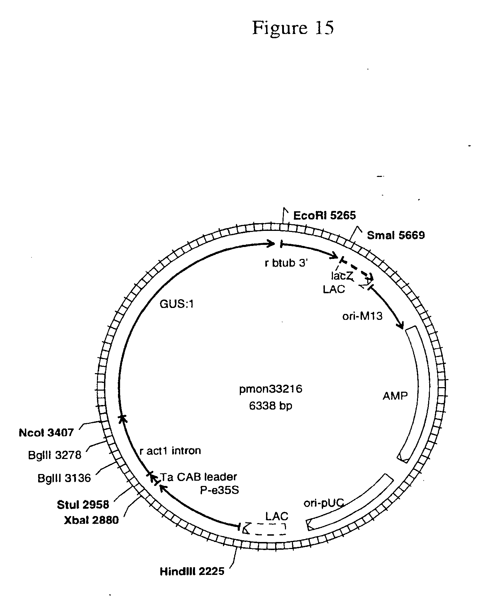

The 3' non-translated terminator from rice lactate dehydrogenase (3' r lacd) (Genebank Accession Number D13817.gb_pl) (SEQ ID NO:62) was isolated by PCR of rice genomic DNA. Amplification was performed using primer sequences SEQ ID NO:37 and SEQ ID NO:38. The DNA was denatured for 1 minute at 95 °C, annealed for 2 minutes at 40 °C, and extended for 3 minutes at 72 °C for a total of 30 cycles. The resultant PCR product was digested with SphI and EcoRI. PMON33218 (Figure 15) contains the inserted SphI / EcoRI fragment.

The 3' non-translated terminator from rice beta-tubulin (3' r btub) (Genebank Accession Number L19598.gb_pl) (SEQ ID NO:63) was isolated by PCR of rice genomic DNA. Amplification was performed using primer sequences SEQ ID NO:39 and SEQ ID NO:40. The DNA was denatured for 1 minute at 95 °C, annealed for 2 minutes at 40 °C, and extended for 3 minutes at 72 °C for a total of 30 cycles. The resultant PCR product was digested with SphI and EcoRI. pMON33216 (Figure 13) contains the inserted SphI / EcoRI fragment.

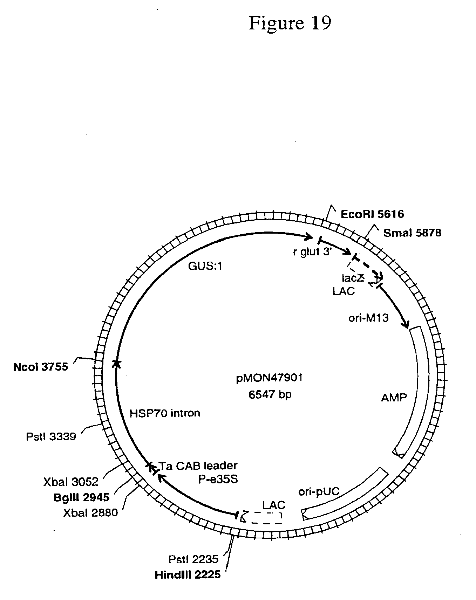

PMON47901 (Figure 19) was constructed by ligating the 3.166 kb EcoRI and PstI fragment of pMON33225, the 0.71 kb PstI and BglII fragment of pMON26038. and the 2.671 kb BglII and EcoRI fragment of pMON19433.

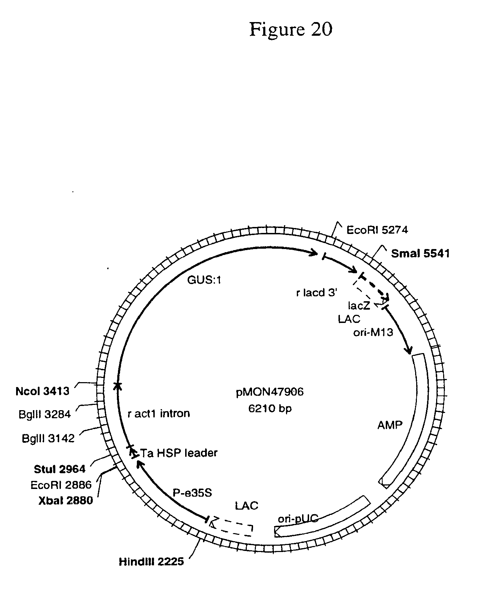

PMON47906 (Figure 20) was constructed by ligating the 5.748 kb NcoI and BglII fragment of pMON47909 and the 0.449 kb StuI and Ncol fragment of pMON33216 using adaptors to the Stul site which create a BglII complementary end (SEQ ID NO. 3 and SEQ ID NO. 4).

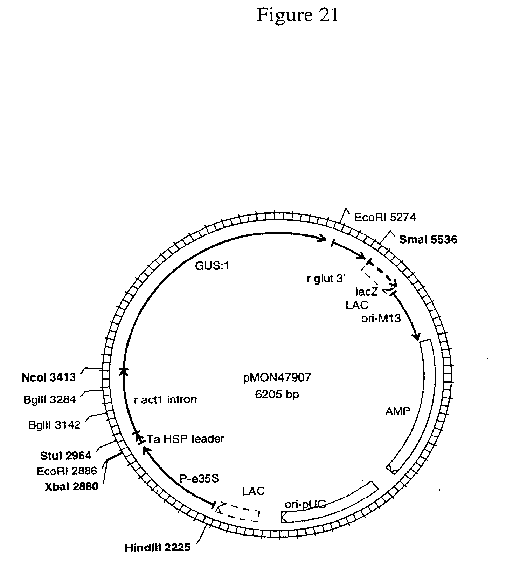

PMON47907 (Figure 21) was constructed by ligating the 5.743 kb NcoI and BglII fragment of pMON47910 and the 0.449 kb Stul and NcoI fragment of pMON33216 using adaptors to the Stul site which create a BglII complementary end (SEQ ID NO. 3 and SEQ ID NO. 4).

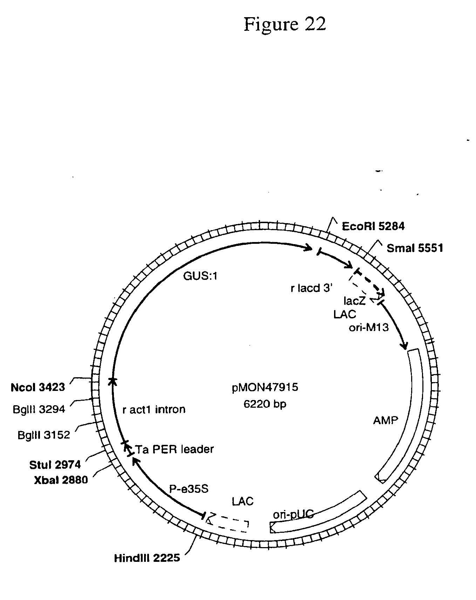

PMON47915 (Figure 22) was constructed by ligating the 5.758 kb NcoI and BglII fragment of pMON47918 8 and the 0.449 kb StuI and Ncol fragment of pMON33216 using adaptors to the Stul site which create a BglII complementary end (SEQ ID NO. 3 and SEQ ID NO. 4).

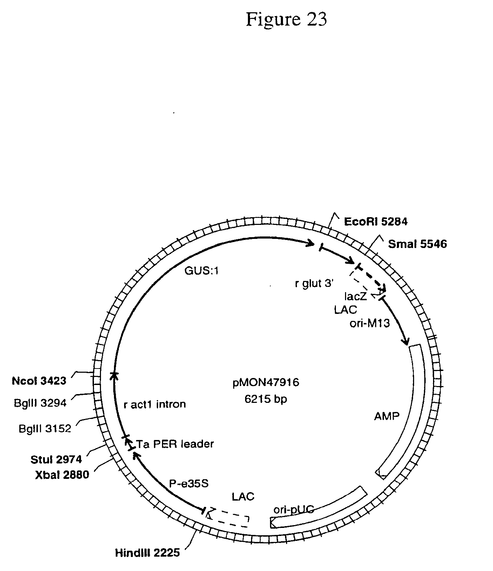

PMON47916 (Figure 23) was constructed by ligating the 5.753 kb Ncol and BglII fragment of pMON47919 9 (Figure 26) and the 0.449 kb StuI and NcoI fragment of pMON33216 using adaptors to the StuI site which create a BglII complementary end (SEQ ID NO. 3 and SEQ ID NO. 4).

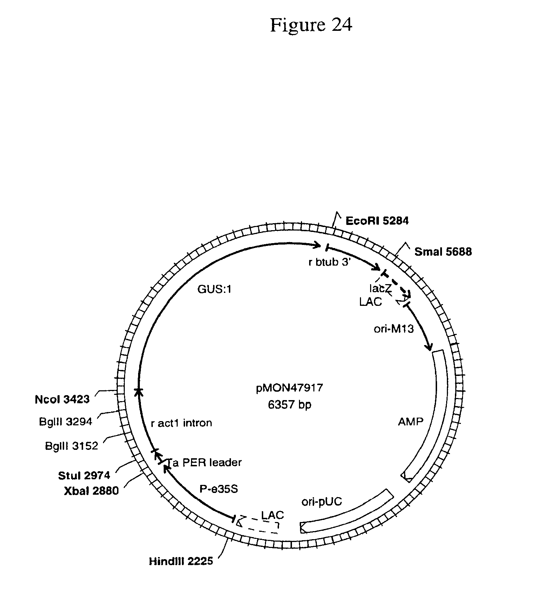

PMON47917 7 (Figure 24) was constructed by ligating the 5.895 kb Ncol and BglII fragment of pMON47920 and the 0.449 kb StuI and NcoI fragment of pMON33216 using adaptors to the StuI site which create a BglII complementary end (SEQ ID NO. 3 and SEQ ID NO. 4).

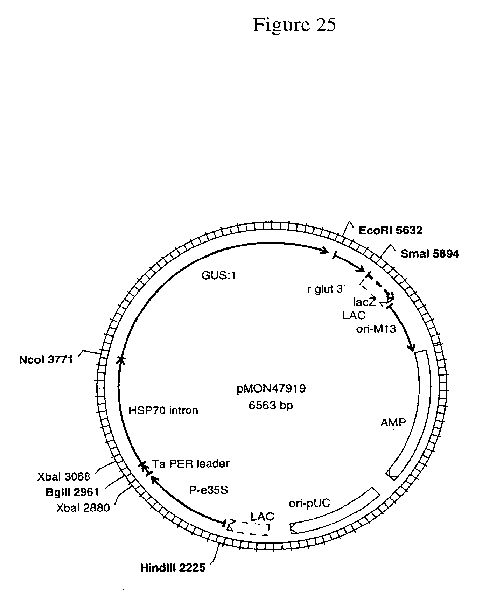

PMON47919 (Figure 25) was constructed by ligating the 3.166 kb EcoRI and Pstl fragment of pMON33225, the 0.726 kb Pstl and BglII fragment of pMON26046, and the 2.671 kb BglII and EcoRI fragment of pMON19433 (Figure 5).

All plasmids were verified by restriction digestion with BglII and were tested in transient expression assays in protoplasts derived from wheat Mustang callus or protoplasts derived from maize BMS callus, or were tested as stably integrated constructs in transgenic plants as described below.

### EXAMPLE 2

### Transient Transformations and Reporter Gene Expression in Wheat and Maize

Purified plasmid DNA was prepared by the Qiagen maxi prep procedure according to the specifications of the manufacturer (Qiagen, Valencia, CA). The GUS plasmids containing 5' non-translated leaders or 3' non-translated terminator regions and combinations of 5' and 3' non-translated regions in vector DNAs and pMON19437 as the internal luciferase control were mixed and electroporated. For reverse experiments to examine enhancements with additional coding sequences, luciferase was the variable reporter gene, GUS in pMON19469 was the internal control. Transformations were performed in duplicate. The 3' non-translated region elements in combination with 5' non-translated leader sequences were also tested relative to the control base vector pMON19469.

Expression analysis of genes in plants has been well documented (Schledzewski et al., 1994; Steinbiss et al., 1991; Stefanov et al., 1991). Protoplast expression analysis such as that described here is often predictive of the expression performance of a recombinant gene in plant cells.

### Analysis of gene expression in wheat protoplasts

The method used for the isolation and preparation of wheat protoplasts was performed as described by Zhou et al., 1993. The electroporation buffer used was described previously (Li et al.. 1995). The culture media used was MS1 WSM (4.4 g Gibco MS salts/L, 1.25 ml Thiamine HCL (0.4mg/ml,), 1 ml 2,4-D (1 mg/ml), 20 g/L sucrose, 0.15 ml asparagine (15 mg/ml), 0.75 g MgCl₂. 109 g.L 0.6 M mannitol, pH5.5.

Mustang suspensions were used for protoplast isolation about four days after subculture. Briefly, 8g of wheat cell suspension was poured into a culture tube and the cells were allowed to settle. The medium was removed, and remaining cells resuspended with 40 ml enzyme solution, transferred to a petri plate, wrapped in foil, and incubated at 26°C for 2 hours on a rotator at 40 rpm. The suspension was centrifuged at 200g for 8 min, washed twice with centrifugation between each wash, resuspended in 10 ml wash solution and stored on ice. The number of protoplasts was determined and the volume adjusted for a final concentration of 4x10⁶ protoplasts/ml. About 0.75 ml of protoplasts was added to each electroporation cuvette and up to about 50 µg plasmid DNA in 50 µl solution was added to the protoplasts. The electroporation conditions were 960 µFarads and 160 volts using a Bio-Rad Gene Pulser (Bio-Rad Laboratories. Hercules. CA). The samples remained on ice for 10 minutes prior to and during the electroporation. After the electroporation, the samples were left on ice for about 10 minutes and then removed and allowed to warm to room temperature for about 10 minutes. The electroporated cells were then pipetted into MS1 WSM media and incubated in the dark for 18-22 hours at 24°C. The cells were harvested by centrifugation at 200-250g for 8 min. and frozen on dry ice for subsequent analysis for the gene(s) of interest.

### Analysis of Gene Expression in Corn Protoplasts

A corn protoplast transient assay system was used to evaluate GUS/LUX expression of the various constructs. Corn leaf protoplast isolation and electroporation was performed as described by Sheen, 1991 with the following changes: the seeds were surface sterilized, germinated in 1/2MS media (2.2 g /L MS salts, 0.25% gelrite), and grown 5 days at 26°C in 16/8 day/night photoperiod, 6 days complete dark, 26°C and 24 hours under the first treatment conditions. The second true leaf from each plant was sliced longitudinally and digested for about 2 hours in the light at 26°C. After digestion, the plates were swirled at 80-100 rpm for 20-30 seconds, and the protoplasts/enzyme solution pipetted through a 190µm tissue collector. The protoplasts were counted using a hemacytometer. Bio-Rad Gene pulser cuvettes (Bio-Rad, Hercules, CA) with a 0.4 cm gap and maximum volume of 0.8 ml were used for the electroporations. Ten to 100 µG of plasmid DNA in addition to 5 µg of DNA containing the luciferase gene as an internal control was added to the cuvette. Final protoplast densities were about 3 million per ml to 4.5 million per ml, with electroporation settings at a 125 µFarad capacitance and 200 volts. Protoplasts were incubated on ice after resuspension in electroporation buffer and remained on ice until 10 minutes after electroporation. The protoplasts were added to about 7 mls of modified MS medium as described in Fromm et al., 1987, with the addition of 0.6M mannitol in petri plates layered with the same media plus 1.5% SeqPlaque agarose (FMC Bioproducts, Rockland, ME)). The protoplasts were harvested by centrifugation 24 hours post-electroporation and used for subsequent expression analysis for the gene(s) of interest.

### GUS activity

GUS (β-glucuronidase) activity was determined from 25 µl of cell extract according to the methods of Jefferson et al. (1987) using 2 mM MUG (4-methylumbelliferyl-β-D-glucuronide) in the previously described extraction buffer. Fluorescence was measured using a Hoescht DNA Fluorometer (Model TKO 100). A methylumbelliferone (Sigma) standard curve was generated using a 1 µM solution.

### Luciferase activity

To determine luciferase activity, ten microliters of each crude test protein extract was dispensed into a microtiter plate. Twenty-five microliters of 2X buffer (50mM Tricine (pH 7.8), 30 mM MgCl₂, 10 mM ATP, and 0.5 mg/mL) was added to each well containing extract. The reactions were initiated by the addition of 25 µl of 10 mM luciferin. The samples were mixed, and the chemiluninescence of each sample was quantitiated on a Packard TopoCount microplate scintillation counter using a 5 minute count delay and a count time of 0.2 minutes.

Results are expressed as a ratio of experimental reporter gene levels to internal control reporter gene levels. The control plasmid contained a different reporter gene and was used to correct for variability in the transformation and extraction procedures.

**TABLE 2 Effect of 5' Non-translated Leaders on GUS Expression in Wheat Mustang Protoplasts**

| Vector | 5' non-translated leader | intron | 3' non-translated terminator | Relative GUS/LUX Expression |
|---|---|---|---|---|
| pMON19469 | base-synthetic | hsp 70 | nos | 1.0 |
| pMON25456 | base-synthetic | rice actin | nos | 0.9 |
| pMON26052 | Ta hsp | hsp 70 | nos | 1.8 |
| pMON26055 | Ta hsp | rice actin | nos | 4.0 |
| pMON26064 | Ta fbp | rice actin | nos | 4.2 |
| pMON26044 | Ta cab | rice actin | nos | 6.7 |

The effect of 5'non-translated leaders on GUS expression in wheat Mustang Protoplasts was measured using constructs which contained various 5' non-translated leaders. The level of expression for the control plasmid containing the base-synthetic 5' sequence (SEQ ID NO:45) the hsp70 intron (SEQ ID NO:47), and the 3' nos terminator region (SEQ ID NO:46) was set as 1.0. GUS expression was increased in the wheat protoplasts when using the wheat heat shock protein (Ta hsp), wheat fructose-1,6-bisphosphatase(Ta fbp), or wheat chlorophyll a/b-binding protein (Ta cab) 5'non-translated leader sequences compared with the base synthetic sequences (Table 2). The effect was less pronounced in maize BMS protoplasts (Table 3).

**TABLE 3 Effect of 5' Non-translated Leaders on GUS Expression in Maize BMS Protoplasts**

| Vector | Leader | intron | 3' terminator | Relative GUS/LUX Expression |
|---|---|---|---|---|
| pMON19469 | base-synthetic | hsp 70 | nos | 1.0 |
| pMON26052 | Ta hsp | hsp 70 | nos | 0.8 |
| pMON26055 | Ta hsp | rice actin | nos | 4.9 |
| pMON26064 | Ta fbp | rice actin | nos | 0.7 |
| pMON26044 | Ta cab | rice actin | nos | ND |

Table 4 shows GUS results in wheat Mustang protoplasts using constructs which contain 3' non-translated sequences from wheat heat shock protein (3' Ta hsp), wheat fructose-1,6-bisphosphatase (3' Ta fbp), or wheat ubiquitin (3' Ta ubiq) in comparison to the vector containing the nos 3' region. Each of the 3' non-translated regions provided increased GUS expression relative to that observed with the nos 3' non-translated region.

**TABLE 4 Effect of 3' Non-translated Region Terminators on GUS Expression in Bombarded Wheat Leaves**

| Vector | Leader | intron | 3' terminator | Relative GUS/LUX Expression |
|---|---|---|---|---|
| pMON19433 | base-synthetic | hsp 70 | nos | 1.0 |
| pMON 18379 | base synthetic | hsp 70 | Ta fbp | 1.9 |
| pMON18375 | base synthetic | hsp 70 | Ta hsp | 2.8 |
| pMON18377 | base synthetic | hsp 70 | Ta ubiq | 2.6 |

The combinatorial effects of the disclosed 5' and 3' non-translated sequences were evaluated in wheat Mustang protoplasts. LUX expression was measured in addition to GUS expression to confirm that increased expression levels were not GUS-specific. Both GUS and LUX expression levels were increased when using the constructs containing the Ta cab. Ta hsp, or Ta fbp 5' non-translated leaders and Ta ubiq, Ta fbp, or Ta hsp 3' non-translated terminators (Table 5).

**TABLE 5 Combinatorial Effects of 5' and 3' Non-translated Terminator Sequences on GUS and LUX Expression in Wheat Mustang Protoplasts**

| Vector | Leader | intron | 3' terminator | Relative GUS/LUX Expression | Relative LUX/GUS Expression |
|---|---|---|---|---|---|
| pMON19469 | base synthetic | maize hsp 70 | nos | 1.0 | -- |
| pMON32502 | Ta cab | rice act | Ta hsp | 11.9 | -- |
| pMON32506 | Ta hsp | rice act | Ta hsp | 1.6 | -- |
| pMON32509 | Ta fbp | rice act | Ta ubiq | 8.5 | -- |
| pMON32510 | Ta hsp | rice act | Ta ubiq | 3.5 | -- |
| pMON32513 | Ta fbp | rice act | Ta fbp | 12.5 | -- |
| pMON19437 | base synthetic | maize hsp 70 | nos | -- | 1.0 |
| pMON32516 | Ta fbp | rice act | Ta ubiq | -- | 6.2 |
| pMON32517 | Ta fbp | rice act | Ta fbp | -- | 6.2 |
| pMON32515 | Ta cab | rice act | Ta hsp | -- | 7.6 |

The combined effects of the 5' non-translated leader sequences and 3' non-translated terminator sequences was also measured in maize BMS protoplasts. GUS expression was generally increased over the base control (Table 6). The results observed in maize corroborate those found in wheat, demonstrating that the beneficial effects of the 5' and 3' non-translated sequences of the invention are not limited to the species from which the non-translated sequences are derived.

**TABLE 6 Combinatorial Effects of 5' and 3' Non-translated Terminator Sequences on GUS Expression in Maize BMS Protoplasts**

| Vector | Leader | intron | 3' terminator | Relative GUS/LUX Expression |
|---|---|---|---|---|
| pMON 19469 | base-synthetic | hsp 70 | nos | 1.0 |
| pMON32502 | Ta cab | rice act | Ta hsp | 5.0 |
| pMON32506 | Ta hsp | rice act | Ta hsp | 5.8 |
| pMON32509 | Ta fbp | rice act | Ta ubiq | 0.9 |
| pMON32510 | Ta hsp | rice act | Ta ubiq | 2.5 |
| pMON32513 | Ta fbp | rice act | Ta fbp | 1.2 |

### EXAMPLE 3 Stable Transformation in Wheat Plants

The effects of the 5'and 3' non-translated sequences on GUS expression were also evaluated in transgenic wheat plants. The procedure for wheat transformation and regeneration was as described in U. S. Patent 5,631,152, herein incorporated by reference, but was modified for G418 selection. In brief, immature embryos were cultured on CM4C medium for 0-4 days (CM4C components: 4.3 g/L Gibco MS salts, 10 ml/L MS vitamins (100X), 0.5 ml/L 2,4-D, 40 g/L maltose, 0.5 g/L Glutamine, 0.75 g/L magnesium chloride, 0.1 g/L casein hydrolysate, 1.95 g/L MES, 2 g/L Phytagel); cultures were transferred to CM4C Raff/mann medium for about 4 days, bombarded and transferred to CM4C containinng 25 mg/L G418 for about 5 days; cultures were regenerated on MMS0.2C containing G418 (25 mg/L) for about 19 days, regenerated on MMS0C containing 25 mg/L G418 for about 33 days and rooted on MMS0C containing 25 mg/L G418 for about 57 days and subsequently transferred to soil at about 75 days. CM4C (G418) media contained 2.2 ml/L of 1 mg/ml pichloram, 1 ml/L G418 (25 mg/ml) and 2 ml/L ascorbic acid (50 mg/ml stock). CM4C Raff/Mann 0.25 contained the following components: 4.4 g/L MS salts, 10 ml/L MS Vitamins (100X), 0.5 ml/L 2,4-D, 40 g/L maltose, 74.3 g/L raffinose, 22.78 g/L mannitol, 0.5 g/L glutamine, 0.75 g/L magnesium chloride, 1.95 g/L MES, 0.1 g/L casein hydrolysate, 2 g/L Phytagel, 2.2 ml pichloram (1 mg/ml) and 2 ml ascorbic acid (50 mg/ml). MMS0.2C media contained 4.3 g/L MS salts. 1.95 g/L MES, 2 ml/L MMS vitamins, 0.2 ml/L 2,4-D, 40 g/L maltose, and 2 g/L agar (Schweizer Hall). MM20.2C (G418) contained 1 ml of 25 mg/ml G418 and 2 ml of 50 mg/ml ascorbic acid. MMS0C contained 4.3 g/L MS salts, 1.95 g/L MES, 2.0 ml/L MMS vitamins, and 40 g/L maltose. MMS0C (G418) contained an addtional 1 ml of 25 mg/ml G418 and 2 ml of 50 mg/ml ascorbic acid. Transgenic lines were established using the DNA constructs described in Table 7 below and plants were evaluated for GUS activity levels.

Relative GUS levels were comparable or greater than the base control vector for many of the constructs containing the non-translated elements of the invention. In particular, constructs containing a Ta cab or Ta fbp 5' non-translated leader sequence used in combination with a Ta hsp or Ta fbp 3'non-translated terminator sequence, provided the highest expression levels. Another preferred construct contained a Ta fbp 5'non-translated leader and a 3'non-translated nos terminator region.

**TABLE 7 Effect of 5' Non-translated Leader and 3' Non-translated Terminator Genetic Elements on Stable GUS Expression in Transgenic Wheat Plants**

| construct | Leader/intron/3' non-translated | n | mean GUS activity | relative mean value | GUS activity range low - high | relative high value |
|---|---|---|---|---|---|---|
| pMON26044 | Ta cab/rice act/nos | 5 | 20.7 ± 28.1 | 0.7 | 0.6 - 72.7 | 1.0 |
| pMON26052 | Ta hsp/hsp 70/nos | 9 | 11.1 ± 9.6 | 0.4 | 0.7 - 26.3 | 0.4 |
| pMON26055 | Ta hsp/rice act/nos | 4 | 15.3 ± 24.1 | 0.6 | 0.6 - 57.0 | 0.8 |
| pMON26064 | Ta fbp/rice act/nos | 4 | 72.1 ± 102.3 | 2.6 | 1.0 - 248.6 | 3.5 |
| pMON32502 | Ta cab/rice act/Ta hsp | 55 | 82.8 ± 135.6 | 3.0 | 0.6 - 779.8 | 11.0 |
| pMON32509 | Ta tbp/rice act/Ta ubiq | 6 | 25.6 ± 24.0 | 0.9 | 1.7 - 71.2 | 1.0 |
| pMON32513 | Ta fbp/rice act/Ta fbp | 10 | 86.1 ± 62.6 | 3.1 | 17.0 - 244.8 | 3.4 |

### All GUS activity values expressed as pmol/min/mg protein n - number of independent wheat plants assayed

The percentage of GUS positive events in transgenic wheat plants was used to determine the effect of 5' non-translated leader and 3' non-translated terminator sequences on recovery of stable GUS expression. Constructs which provided high expression levels of GUS in Table 7 also caused a high percentage of GUS positive events (Table 8).

**TABLE 8 Effect of 5' Non-translated Leader and 3' Non-translated Terminator Genetic Elements on Recovery of Stable GUS Expression Above Background Threshold Levels in Transgenic Wheat Plants**

| Construct | Leader/intron/3' non-translated | Number plants | Number events | GUS positive events | % GUS positive events |
|---|---|---|---|---|---|
| pMON19468 | base/hsp 70/nos | 29 | 14 | 2 | 14 |
| pMON26044 | Ta cab/rice act/nos | 17 | 11 | 4 | 36 |
| pMON26052 | Ta hsp/hsp 70/nos | 31 | 22 | 5 | 23 |
| pMON26055 | Ta hsp/rice act/nos | 20 | 15 | 4 | 27 |
| pMON26064 | Ta fbp/rice act/nos | 9 | 8 | 4 | 50 |
| pMON32502 | Ta cab/rice act/Ta hsp | 122 | 58 | 33 | 57 |
| pMON32509 | Ta fbp/rice act/Ta ubiq | 20 | 15 | 4 | 27 |
| pMON32513 | Ta fbp/rice act/Ta fbp | 44 | 37 | 16 | 43 |

**TABLE 9 Effect of 5' Non-translated Leaders on GUS Expression in Maize Leaf Protoplasts**

| Construct | 5' Leader | Intron | 3' Terminator | Relative GUS/LUX |
|---|---|---|---|---|
| pMON8677 | base synthetic | none | nos | 1.0 |
| pMON26038 | Ta cab | none | nos | 12.7 |
| pMON26043 | Ta hsp | none | nos | 7.1 |
| pMON26046 | Ta hsp | none | nos | 6.2 |
| pMON33219 | r amyl | none | nos | 1.7 |

**TABLE 10 Effect of 5' Non-translated Leaders on GUS Expression in Maize Leaf Protoplasts**

| Construct | 5' non-translated leader | Intron | 3' non-translated terminator | Relative GUS/LUX Expression |
|---|---|---|---|---|
| pMON33210 | base synthetic | hsp70 | nos | 1.0 |
| pMON33220 | r btub | hsp70 | nos | 1.5 |

The effect of various 5' non-translated leaders on GUS expression in maize leaf protoplasts was measured using constructs which contained various 5' non-translated leaders (Tables 9 and 10). GUS expression was increased in maize leaf protoplasts when using the wheat chlorophyll a/b-binding protein (Ta cab), wheat heat shock protein (Ta hsp), wheat peroxidase (Ta per), or rice beta-tubulin (r btub) 5' non-translated leader sequences.

**TABLE 11 Effect of Introns on GUS Expression in Maize Leaf Protoplasts**

| Construct | 5' non-translated leader | Intron | 3' non-translated terminator | Relative GUS/LUX Expression |
|---|---|---|---|---|
| pMON8677 | base synthetic | none | nos | 1.0 |
| pMON3321 | base synthetic | r amyl | nos | 5.6 |
| pMON3226 | base synthetic | r pal | nos | 2.5 |
| pMON3228 | base synthetic | r ss1 | nos | 2.3 |

The effect of various introns on GUS expression in maize leaf protoplasts was measured using constructs which contained various introns. GUS expression was increased in maize leaf protoplasts when using the first amylase (r amyl), the phenylalanine ammonia- lyase (r pal), or the first sucrose synthase (ss1) introns from rice.

**TABLE 12 Effect of 3' Non-translated Region Terminators on GUS Expression in Maize Leaf Protoplasts**

| Construct | 5' non-translated leader | Intron | 3' non-translated terminator | Relative GUS/LUX Expression |
|---|---|---|---|---|
| pMON26044 | Ta cab | rice actin | nos | 1.0 |
| pMON33225 | Ta cab | rice actin | r glut | 4.0 |
| pMON33218 | Ta cab | rice actin | r lacd | 4.5 |
| pMON33216 | Ta cab | rice actin | r btub | 3.6 |

The effect of various 3' non-translated terminators on GUS expression in maize leaf protoplasts was measured using constructs which contained various 3' non-translated terminators. GUS expression was increased in maize leaf protoplasts using the rice glutelin type II (r glut), rice lactate dehydrogenase (r laed), or the rice beta-tubulin (r btub) 3' non-translated terminators compared to the control construct which contained the nos 3' non-translated terminator.

**TABLE 13 Combinatorial Effects of 5' and 3' Non-translated Sequences on GUS Expression in Maize Leaf Protoplasts**

| Construct | 5' non-translated leader | Intron | 3' non-translated terminator | Relative GUS/LUX Expression |
|---|---|---|---|---|
| pMON19469 | base synthetic | hsp70 | nos | 1.0 |
| pMON33218 | Ta cab | rice actin | r lacd | 2.8 |
| pMON33225 | Ta cab | rice actin | r glut | 3.1 |
| pMON47901 | Ta cab | rice actin | r glut | 2.7 |
| pMON47906 | Ta hsp | rice actin | r lacd | 3.2 |
| pMON47907 | Ta hsp | rice actin | r glut | 3.5 |
| pMON47915 | Ta per | rice actin | r lacd | 3.4 |
| pMON47916 | Ta per | rice actin | r glut | 3.9 |
| pMON47919 | Ta per | hsp70 | r btub | 3.2 |
| pMON47917 | Ta per | rice actin | r btub | 2.4 |
| pMON32502 | Ta cab | rice actin | Ta hsp | 3.0 |

**TABLE 14 Effects of Various Leader Sequences on GUS Expression in Maize Leaf Protoplasts**

| Construct | 5' non-translated leader | Intron | 3' non-translated leader | Relative GUS/LUX Expression |
|---|---|---|---|---|
| pMON8677 | none | hsp70 | nos | 1.0 |
| pMON33219 | ramy | hsp70 | nos | 1.65 |
| pMON26038 | Ta cab | none | nos | 12.74 |
| pMON26043 | Ta hsp | none | nos | 7.11 |
| pMON26046 | Ta per | none | nos | 6.16 |
| pMON33210 | none | hsp70 | nos | 1.0 |

### REFERENCES

Bird and Ray, Biotechnology and Genetic Engineering Reviews 9: 207-27 (1991)
Birren et al., Genome Analysis: Analyzing DNA, 1, Cold Spring Harbor , New York (1996)
Bouhida et al.. Journal General Virology (1993) vol. 74 pp. 15-22.
Callis et al., Genes and Develop. 1:1183-1200 (1987)
Carrington and Freed, J. of Vir. 64, 1590-1597 (1990)
Coruzzi et al. EMBO J. (1984) vol. 3 pp. 1671-1679.
Dellaporta et al., Stadler Symposium 11:263-282 (1988)
Dietrich et al., J. Cell Biol. 105, 67, (1987)
Fromm et al. Methods Enzymol. 153, 351-366 (1987)
Gallie et al., NAR 15, 8693-8711 (1987)
Gallie et al., The Plant Cell 1:301-311 (1989)
Gelvin et al., In: Plant Molecular Biology Manual, Kluwer Academic Publishers (1990)
Gibson and Shillitoe, Molecular Biotech. 7(2) 125-37 (1997)
Goff, et al.. EMBO J. 9: 2517-2522 (1990)
Hattori, et al., Genes Dev. 6: 609-618 (1992)
Hinchee et al., Bio/Technology 6:915-922 (1988)
Hinchee et al., Plant Transformation, In PLANT CELL AND TISSUE CULTURE, 231-270, 1994, Vasil and Thorpe (Eds.), Dordrecht Publishing, Netherlands.
Ikatu et al., Bio/Technol. 8:241-242 (1990)
Innes et al., In: PCR Protocols, A Guide to Methods and Applications, Academic Press, San Diego, 1990
Jackson et al., TIBS 15, 477-483 (1990)
Jefferson et al., EMBO J. 6: 3901-3907 (1987)
Jefferson, Plant Mol. Biol, Rep. 5: 387-405 (1987)
Jobling and Gehrke, Nature 325, 622-625 (1987)
Jorgensen, Trends in Biotech 8(12) 340-44 (1990)
Joshi, Nucl Acids Res 15: 6643-6653, 1987
Katz et al., J. Gen. Microbiol. 129:2703-2714 (1983)
Klein et al.. Bio/Technology 6:559-563.
Kozak. Cell 44. 283-292 (1986)
Kozak, Mol. and Cell. Biol. 8, 2737-2744 (1988)
Koziel et al., Plant Mol. Biol. 32:393-405 (1996)
Li et al. Molecular Breeding (1997) vol. 3 pp. 1-14.
Mailga et al., Methods in Plant Molecular Biology, Cold Spring Harbor Press (1995)
Marcotte, et al., Nature, 335: 454-457 (1988)
Marcotte, et al., Plant Cell, 1: 523-532 (1989)
Mascarenkas et al., Plant Mol. Biol., 15, 913-920 (1990)
McCarty, et al., Cell 66: 895-905 (1991)
McElroy et. al., Plant Cell. 2:163-71 (1990)
McElroy et al., Mol. Gen. Genet. 231:150-160 (1991)
Medberry and Olszewski. Plant Journal (1993) vol. 3 pp. 619-626.
Moffatt et al. Gene (1994) vol 143 pp. 211-216.
Moldave, Ann. Rev. Biochem.54,1109-1149 (1985)
Ni et al. Plant J. (1995) vol. 7 pp. 661-676.
Odell et al. Nature (1985) vol. 313 pp. 810-812.
Ow et al., Science 234: 856-859 (1986)
Pain, Biochem. J. 235, 625-637 (1986)
Pelletier and Sonenberg, Cell 40, 515-526 (1985)
Potrykus et al., Mol. Gen. Genet. 199:183-188 (1985)
Pouwels et al.. In Cloning Vectors. A Laboratory Manual, 1985, supp., 1987
Ritchie et al.. In TRANSGENIC PLANTS, Vol. 1, 147-177, 1993, Kung and Wu (Eds.), Academic Press
Inc, Harcourt Brace Jovanovich Publishers.
Sambrook et al., Molecular Cloning: A Laboratory Manual, Cold Spring Harbor Press (1989)
Sanger et al. Plant Mol. Biol. (1990) vol. 14 pp. 433-443
Schledzewski et al., Transgenic Research (1994) 3: 249-255
Schuch, Symposia of the Society for Experimental Biology 45: 117-27 (1991)
Sheen et al. Plant Cell 3(3) 225-245 (1991)
Skuzeski et al., Plant mol. Biol. 15. 65-79 (1990)
Sonenberg, Curr. Top. Micro. and Imm. 161, 23-47 (1990)
Songstad et al., In Vitro Cell. Dev. Biol. Plant 32:179-183 (1995)
Stalker et al., J. Biol. Chem. 263:6310-6314 (1988)
Stefanov et al., Acta Biologica Hungarica (1991) 42: 323-330
Steinbiss et al., Subcellular Biochem. (1991) 17: 143-166
Sutcliffe et al., Proc. Natl. Acad. Sci. (U.S.A.) 75: 3737-3741 (1978)
Tanaka, Nucl. Acids Res. 18:6767-6770, 1990
Thillet et al., J. Biol. Chem. 263:12500-12508 (1988)
Vasil et al., Bio/Technology 10:667, (1992)
Wang et al., Plant Mol. Biol., 19:881-885 (1992)
Weissbach and Weisbach, In Methods for Plant Molecular Biology, Academic Press. 1989
Wen et al. Chinese J. of Bot. (1993) vol. 5 pp. 102-109
Xu et al. Plant Physiology (1994) vol. 106 pp. 459-467
Zhong et al. Plant Science (1996) vol. 116 pp. 73-84
Zhou. H. et al., Plant Cell Reports (1993) 12: 612-616
Zhu et al., Plant Mol. Biol. 29:535-550 (1995)
Zukowsky et al., Proc. Natl. Acad. Sci. (U.S.A.) 80:1101-1105 (1983)

## Claims

1. A recombinant DNA molecule which comprises, operably linked in the 5' to 3' direction,
(a) a promoter sequence;
(b) a 5' non-translated leader sequence comprising SEQ ID NO:53;
(c) a first intron sequence isolated from a nucleotide sequence associated with a gene selected from a rice actin gene, a rice sucrose synthase gene, a rice phenylalanine ammonia lyase gene, a maize heat shock protein gene and a rice amylase gene;
(d) a DNA coding sequence; and
(e) a 3' non-translated terminator sequence isolated from a nucleotide sequence associated with a gene selected from a wheat heat shock protein gene, a wheat ubiquitin gene, a wheat fructose-1,6-bisphosphatase gene, a rice glutelin gene, a rice lactate dehydrogenase gene, and a rice beta-tubulin gene.

2. The DNA molecule of claim 1, wherein the first intron sequence is isolated from
(i) a nucleotide sequence associated with a rice actin gene; or
(ii) a nucleotide sequence associated with a rice sucrose synthase gene; or
(iii) a nucleotide sequence associated with a rice phenylalanine ammonia lyase gene; or
(iv) a nucleotide sequence associated with a rice amylase gene.

3. The DNA molecule of claim 1, wherein the first intron sequence is isolated from a nucleotide sequence associated with a rice actin gene, and the 3' non-translated terminator sequence is isolated from a nucleotide sequence associated with a wheat heat shock protein gene or a nucleotide sequence associated with a wheat fructose-1,6-bisphosphatase gene.

4. The DNA molecule of claim 1, wherein
(i) the promoter is constitutive, inducible, developmentally regulated, chemically regulated, tissue-enhanced, or tissue specific; or
(ii) the DNA coding sequence is in the sense orientation; or
(iii) the DNA coding sequence is in the antisense orientation.

5. A transformed cell comprising a recombinant DNA molecule according to claim 1.

6. The transformed cell of claim 5, wherein the cell is a plant or bacterial cell.

7. A plant comprising the plant cell of claim 6.

8. The plant of claim 7, wherein the plant is a monocot.

9. The plant of claim 7, which is selected from barley, oat, corn, rice, rye, and wheat.

10. A method for providing enhanced gene expression in monocotyledonous plants which comprises:
(a) transforming monocotyledonous plant cells with a recombinant DNA molecule according to claim 1;
(b) selecting plant cells which have been transformed; and,
(c) regenerating the selected plant cells to provide a differentiated plant.

11. The method of claim 10, wherein the first intron sequence is isolated from
(i) a nucleotide sequence associated with a rice actin gene; or
(ii) a nucleotide sequence associated with a rice sucrose synthase gene; or
(iii) a nucleotide sequence associated with a rice phenylalanine ammonia lyase gene.

12. The method of claim 10, wherein the recombinant DNA molecule comprises a first intron sequence isolated from a nucleotide sequence associated with a rice actin gene, and a 3' non-translated terminator sequence isolated from a nucleotide sequence associated with a wheat heat shock protein gene, preferably the recombinant DNA molecule comprises a first intron sequence comprising SEQ ID NO:50, and a 3' non-translated terminator sequence comprising SEQ ID NO:58.

13. The method of claim 10, wherein the recombinant DNA molecule comprises a first intron sequence isolated from a nucleotide sequence associated with a rice actin gene, and a 3' non-translated terminator sequence isolated from a nucleotide sequence associated with a wheat fructose-1,6-bisphosphatase gene, preferably the recombinant DNA molecule comprises a first intron sequence comprising SEQ ID NO:50, and a 3' non-translated terminator sequence comprising SEQ ID NO:60.

14. The method of claim 10, wherein the DNA coding sequence
(i) is in the sense orientation; or
(ii) is in the antisense orientation.

15. The method of claim 10, wherein the promoter is constitutive, inducible, developmentally regulated, chemically regulated, tissue-enhanced, or tissue-specific.

## Patentansprüche

1. Rekombinantes DNA-Molekül, das in 5'→3'-Richtung funktionell miteinander verknüpft Folgendes umfasst:
(a) eine Promotorsequenz;
(b) eine 5'-untranslatierte Leitsequenz, die SEQ ID Nr. 53 umfasst;
(c) eine erste Intronsequenz, die aus einer Nucleotidsequenz isoliert ist, welche mit einem Gen assoziiert ist, das aus einem Reis-Actin-Gen, einem Reis-Sucrose-Synthase-Gen, einem Reis-Phenylalanin-Ammoniak-Lyase-Gen, einem Mais-Hitzeschockprotein-Gen und einem Reis-Amylase-Gen ausgewählt ist;
(d) eine DNA-codierende Sequenz; und
(e) eine 3'-untranslatierte Terminatorsequenz, die aus einer Nucleotidsequenz isoliert ist, welche mit einem Gen assoziiert ist, das aus einem Weizen-Hitzeschockprotein-Gen, einem Weizen-Ubiquitin-Gen, einem Weizen-Fructose-1,6-Bisphosphatase-Gen, einem Reis-Glutelin-Gen, einem Reis-Lactat-Dehydrogenase-Gen und einem Reis-β-Tubulin-Gen ausgewählt ist.

2. DNA-Molekül gemäß Anspruch 1, wobei die erste Intronsequenz isoliert ist aus:
(i) einer Nucleotidsequenz, die mit einem Reis-Actin-Gen assoziiert ist; oder
(ii) einer Nucleotidsequenz, die mit einem Reis-Sucrose-Synthase-Gen assoziiert ist; oder
(iii) einer Nucleotidsequenz, die mit einem Reis-Phenylalanin-Ammoniak-Lyase-Gen assoziiert ist; oder
(iv) einer Nucleotidsequenz, die mit einem Reis-Amylase-Gen assoziiert ist.

3. DNA-Molekül gemäß Anspruch 1, wobei die erste Intronsequenz aus einer Nucleotidsequenz isoliert ist, die mit einem Reis-Actin-Gen assoziiert ist, und die 3'-untranslatierte Terminatorsequenz aus einer Nucleotidsequenz, die mit einem Weizen-Hitzeschockprotein-Gen assoziiert ist, oder aus einer Nucleotidsequenz, die mit einem Weizen-Fructose-1,6-Bisphosphatase-Gen assoziiert ist, isoliert ist.

4. DNA-Molekül gemäß Anspruch 1, wobei
(i) der Promotor konstitutiv, induzierbar, entwicklungsmäßig reguliert, chemisch reguliert, gewebeverstärkt oder gewebespezifisch ist; oder
(ii) die DNA-codierende Sequenz in Sense-Orientierung vorliegt; oder
(iii) die DNA-codierende Sequenz in Antisense-Orientierung vorliegt.

5. Transformierte Zelle, die ein rekombinantes DNA-Molekül gemäß Anspruch 1 umfasst.

6. Transformierte Zelle gemäß Anspruch 5, wobei die Zelle eine Pflanzen- oder Bakterienzelle ist.

7. Pflanze, die die Pflanzenzelle gemäß Anspruch 6 umfasst.

8. Pflanze gemäß Anspruch 7, wobei die Pflanze einkeimblättrig ist.

9. Pflanze gemäß Anspruch 7, die aus Gerste, Hafer, Mais, Reis, Roggen und Weizen ausgewählt ist.

10. Verfahren zum Hervorbringen einer verstärkten Gen-Expression in einkeimblättrigen Pflanzen, umfassend:
(a) Transformieren von Zellen einkeimblättriger Pflanzen mit einem rekombinanten DNA-Molekül gemäß Anspruch 1;
(b) Selektieren von Pflanzenzellen, die transformiert wurden; und
(c) Regenerieren der selektierten Pflanzenzellen unter Bildung einer ausdifferenzierten Pflanze.

11. Verfahren gemäß Anspruch 10, wobei die erste Intronsequenz isoliert ist aus:
(i) einer Nucleotidsequenz, die mit einem Reis-Actin-Gen assoziiert ist; oder
(ii) einer Nucleotidsequenz, die mit einem Reis-Sucrose-Synthase-Gen assoziiert ist; oder
(iii) einer Nucleotidsequenz, die mit einem Reis-Phenylalanin-Ammoniak-Lyase-Gen assoziiert ist.

12. Verfahren gemäß Anspruch 10, wobei das rekombinante DNA-Molekül eine erste Intronsequenz, die aus einer mit einem Reis-Actin-Gen assoziierten Nucleotidsequenz isoliert ist, und eine 3'-untranslatierte Terminatorsequenz, die aus einer mit einem Weizen-Hitzeschockprotein-Gen assoziierten Nucleotidsequenz isoliert ist, umfasst, wobei das rekombinante DNA-Molekül vorzugsweise eine erste Intronsequenz, die SEQ ID Nr. 50 umfasst, und eine 3'-untranslatierte Terminatorsequenz, die SEQ ID Nr. 58 umfasst, umfasst.

13. Verfahren gemäß Anspruch 10, wobei das rekombinante DNA-Molekül eine erste Intronsequenz, die aus einer mit einem Reis-Actin-Gen assoziierten Nucleotidsequenz isoliert ist, und eine 3'-untranslatierte Terminatorsequenz, die aus einer mit einem Weizen-Fructose-1,6-Bisphosphatase-Gen assoziierten Nucleotidsequenz isoliert ist, umfasst, wobei das rekombinante DNA-Molekül vorzugsweise eine erste Intronsequenz, die SEQ ID Nr. 50 umfasst, und eine 3'-untranslatierte Terminatorsequenz, die SEQ ID Nr. 60 umfasst, umfasst.

14. Verfahren gemäß Anspruch 10, wobei die DNA-codierende Sequenz
(i) in Sense-Orientierung vorliegt; oder
(ii) in Antisense-Orientierung vorliegt.

15. Verfahren gemäß Anspruch 10, wobei der Promotor konstitutiv, induzierbar, entwicklungsmäßig reguliert, chemisch reguliert, gewebeverstärkt oder gewebespezifisch ist.

## Revendications

1. Molécule d'ADN recombinant qui comprend, liées de manière fonctionnelle dans le sens 5' vers 3',
(a) une séquence promotrice ;
(b) une séquence de tête non traduite en 5' comprenant la séquence SEQ ID NO : 53 ;
(c) une première séquence d'intron isolée à partir d'une séquence nucléotidique associée à un gène choisi parmi un gène d'actine de riz, un gène de saccharose synthase de riz, un gène de phénylalanine ammoniac lyase de riz, un gène de protéine de choc thermique de maïs et un gène d'amylase de riz ;
(d) une séquence codante d'ADN ; et
(e) une séquence de terminaison non traduite en 3' isolée à partir d'une séquence nucléotidique associée à un gène choisi parmi un gène de protéine de choc thermique de blé, un gène d'ubiquitine de blé, un gène de fructose-1,6-bisphosphatase de blé, un gène de glutéline de riz, un gène de lactate déshydrogénase de riz, et un gène de bêta-tubuline de riz.

2. Molécule d'ADN de la revendication 1, dans laquelle la première séquence d'intron est isolée à partir
(i) d'une séquence nucléotidique associée à un gène d'actine de riz ; ou
(ii) d'une séquence nucléotidique associée à un gène de saccharose synthase de riz ; ou
(iii) d'une séquence nucléotidique associée à un gène de phénylalanine ammoniac lyase de riz ; ou
(iv) d'une séquence nucléotidique associée à un gène d'amylase de riz.

3. Molécule d'ADN de la revendication 1, dans laquelle la première séquence d'intron est isolée à partir d'une séquence nucléotidique associée à un gène d'actine de riz, et la séquence de terminaison non traduite en 3' est isolée à partir d'une séquence nucléotidique associée à un gène de protéine de choc thermique de blé ou d'une séquence nucléotidique associée à un gène de fructose-1,6-bisphosphatase de blé.

4. Molécule d'ADN de la revendication 1, dans laquelle
(i) le promoteur est constitutif, inductible, à régulation du stade de développement, à régulation chimique, à expression accrue dans un tissu, ou à spécificité tissulaire ; ou
(ii) la séquence codante d'ADN est dans l'orientation sens ; ou
(iii) la séquence codante d'ADN est dans l'orientation antisens.

5. Cellule transformée comprenant une molécule d'ADN recombinant selon la revendication 1.

6. Cellule transformée de la revendication 5, dans laquelle la cellule est une cellule végétale ou bactérienne.

7. Plante comprenant la cellule végétale de la revendication 6.

8. Plante de la revendication 7, dans laquelle la plante est une monocotylédone.

9. Plante de la revendication 7, qui est choisie parmi l'orge, l'avoine, le maïs, le riz, le seigle, et le blé.

10. Procédé destiné à fournir une expression de gène accrue dans des plantes monocotylédones qui comprend le fait :
(a) de transformer des cellules de plantes monocotylédones avec une molécule d'ADN recombinant selon la revendication 1 ;
(b) de sélectionner des cellules végétales qui ont été transformées ; et,
(c) de régénérer les cellules végétales sélectionnées pour fournir une plante différenciée.

11. Procédé de la revendication 10, dans lequel la première séquence d'intron est isolée à partir
(i) d'une séquence nucléotidique associée à un gène d'actine de riz ; ou
(ii) d'une séquence nucléotidique associée à un gène de saccharose synthase de riz ; ou
(iii) d'une séquence nucléotidique associée à un gène de phénylalanine ammoniac lyase de riz.

12. Procédé de la revendication 10, dans lequel la molécule d'ADN recombinant comprend une première séquence d'intron isolée à partir d'une séquence nucléotidique associée à un gène d'actine de riz, et une séquence de terminaison non traduite en 3' isolée à partir d'une séquence nucléotidique associée à un gène de protéine de choc thermique de blé, de préférence la molécule d'ADN recombinant comprend une première séquence d'intron comprenant la séquence SEQ ID NO : 50, et une séquence de terminaison non traduite en 3' comprenant la séquence SEQ ID NO : 58.

13. Procédé de la revendication 10, dans lequel la molécule d'ADN recombinant comprend une première séquence d'intron isolée à partir d'une séquence nucléotidique associée à un gène d'actine de riz, et une séquence de terminaison non traduite en 3' isolée à partir d'une séquence nucléotidique associée à un gène de fructose-1,6-bisphosphatase de blé, de préférence la molécule d'ADN recombinant comprend une première séquence d'intron comprenant la séquence SEQ ID NO : 50, et une séquence de terminaison non traduite en 3' comprenant la séquence SEQ ID NO : 60.

14. Procédé de la revendication 10, dans lequel la séquence codante d'ADN
(i) est dans l'orientation sens ; ou
(ii) est dans l'orientation antisens.

15. Procédé de la revendication 10, dans lequel le promoteur est constitutif, inductible, à régulation du stade de développement, à régulation chimique, à expression accrue dans un tissu, ou à spécificité tissulaire.
